# EUROPEAN PATENT SPECIFICATION

(11) **EP 3 458 162 B1**
(45) Date of publication and mention of the grant of the patent: **10.07.2024**
(21) Application number: 17724361.5
(22) Date of filing: 19.05.2017
(51) Int. Cl.: A61Q 17/04, A61K 8/06, A61K 8/49, A61K 8/42, A61K 8/81, A61K 8/44, A61K 8/64, A61K 8/365, A61K 8/46, A61K 8/58, A61K 8/25

(54) **MICRO-PARTICULATE ORGANIC UV ABSORBER COMPOSITION**
MIKROPARTIKULÄRE, ORGANISCHE, UV-ABSORBIERENDE ZUSAMMENSETZUNG
COMPOSITION D'ABSORBEUR UV ORGANIQUE MICROPARTICULAIRE

(30) Priority: 19.05.2016 EP 16170307
(43) Date of publication of application: 27.03.2019
(73) Proprietor: BASF SE, 67056 Ludwigshafen am Rhein (DE)
(72) Inventor: FLOESSER-MUELLER, Heike, 67056 Ludwigshafen (DE); ACKER, Stephanie, 79639 Grenzach-Wyhlen (DE); DANOUX, Louis, 54272 Essey-les-Nancy (FR); URCH, Henning, 67117 Limburgerhof (DE); GRUMELARD, Julie, 79639 Grenzach-Wyhlen (DE); EHLIS, Thomas, 4057 Basel (CH); HERZOG, Bernd, 79639 Grenzach-Wyhlen (DE); MENGE, Ullrich, 79639 Grenzach-Wyhlen (DE); YOUNG, Antony, WC2R 2LS London (GB); LAWRENCE, Karl, WC2R 2LS London (GB)
(74) Representative: BASF IP Association
(86) International application number: PCT/EP2017/062075
(87) International publication number: WO 2017/198806

(56) References cited:
- WO-A1-2009/003934
- WO-A1-2016/012586
- WO-A2-2007/071584
- WO-A2-2010/091963
- WO-A2-2011/086073

## Description

Disclosed are aqueous dispersions of micronized organic UV filters and new sunscreen compositions, which comprise aqueous dispersions of micronized organic UV filters.

It has long been known that prolonged exposure to UV radiation can lead to the formation of erythemas or light dermatoses, as well as to an increased incidence of skin cancers, or accelerated skin ageing.

Various sunscreen formulations have been proposed which include materials which are intended to counteract UV radiation, thereby inhibiting the said undesired effects on the skin. A great number of compounds has been proposed for use as UV filters in sunscreen formulations, especially soluble organic UV absorbers and insoluble micronized organic compounds.

Micronized insoluble organic UV absorbers produced according to the said method are used in sunscreen formulations and provide excellent UV protection and have an SPF rating which is at least as high as corresponding sunscreen formulations containing a known inorganic UV absorber.

GB-A-2303549 describes a method of producing micronized, insoluble organic UV absorbers, as well as a sunscreen composition comprising an aqueous dispersion of micronized insoluble organic UV absorbers. The method comprises grinding the insoluble organic UV absorber, in coarse particle form, in a grinding apparatus, in the presence of a polyglucoside. WO 2007 071584 describes suitable anionic, nonionic and amphoteric surfactants with a HLB (Hydrophile-Lipophile Balance) value higher than 8, and preferably higher than 10 as grinding aids for the micronization process the organic UV absorbers are. This reference teaches that a multitude of cosmetic surfactants may be used for the micronization of insoluble organic UV filters, the most preferred dispersants being sodium alkyl sulfates or sodium alkyl ether sulfates. However, these mostly in rinse-off surfactants are not well accepted and irritant to the skin. More preferred are leave-on dispersants, which are mild to the skin.

WO 2009 068469 discloses many exemplary compositions for micronized dispersions. All formulations contain a thickening agent, such as xanthan gum for the prevention of sedimentation of the UV filter particles and thus for the increase of the shelf life of the dispersion. However, the use of xanthan gum requires the preparation of a concentrated thickener dispersion in a suitable solvent as an additional process step. This thickener dispersion is then mixed into the micronized UV filter dispersion in order to achieve a homogenous UV filter dispersion. For this solvent water / glycol mixtures are frequently used. Thus, the use of xanthan gum as thickener for micronized UV filter dispersions may also lead to a certain level of glycol in the sunscreen product which is not always desired in cosmetic formulations.

Thickening agents used in cosmetics include viscous liquids such as polyethylene glycol, synthetic polymers such as carbomer (a trade name for polyacrylic acid) and vegetable gums like xanthan gum. Some thickening agents may also function as stabilizers when they are used to maintain the stability of an emulsion.

Finally, the use of organic micronized filters may offer new benefits to the consumer due to their specific properties. Their UV protection spectrum can be extended to longer wavelengths of the UVA- and even beyond UV light - up to the blue visible light (up to 500 nm) without causing severe whitening of the skin, enabling enhanced and new areas of protection: very long UVA and near VIS light, for example, are known to efficiently induce free radicals in skin [Zastrow 2007]. Furthermore, short wavelength visible light is reported to be involved in specific pigmentation mechanisms like the tanning in darker skin types [Mahmoud 2010], the formation of post-inflammatory hyperpigmentation and of melasma [Castanedo-Cazares JP1 et al; Photodermatol Photoimmunol Photomed. 2014 Feb;30(1):35-42. doi: 10.1111/phpp. 12086. Epub 2013 Dec 3]. Hence, enhanced protection of the skin at longer wavelengths is a very important beneficial effect.

Therefore, the problem of the present invention was to overcome the outlined shortcomings in the formulation of micronized organic UV filters and to provide new benefits to cosmetic and pharmaceutical formulations.

The first aspect of the present invention relates to an aqueous dispersion (B) comprising 20 to 60 % by weight of at least one UV filter selected from
(a₁) the micronized compound of formula 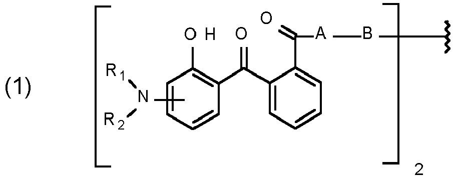 , wherein
   R₁ and R₂ independently from each other are C₁-C₂₀alkyl; C₂-C₂₀alkenyl; C₃-C₁₀cycloalkyl; C₃-C₁₀cycloalkenyl; or R₁ and R₂ together with the linking nitrogen atom form a 5- or 6-membered heterocyclic ring;
   A is --N(R₃)-; -O-; or the direct bond;
   B is a bivalent radical selected from alkylene, cycloalkylene alkenylene or phenylene radical which is optionally substituted by a carbonyl- or carboxy group; a radical of formula *-CH₂C≡C-CH₂-* ; or the divalent radical *-B-* corresponds to the formula 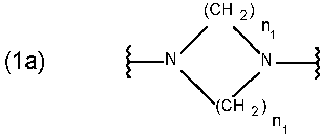 ; wherein
   n₁ is a number from 1 to 3;
   A is --N(R₃)-; or -O-; and
   R₃ is hydrogen; C₁-C₅alkyl; or hydroxy-C₁-C₅alkyl;
(az) the micronized compound of formula 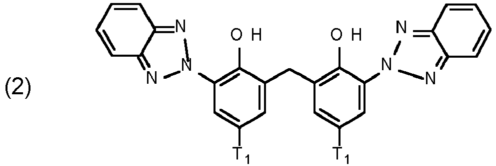 , wherein
   T₁ is hydrogen; C₁-C₁₂alkyl; preferably iso-octyl, or C₁-C₄alkyl substituted by phenyl;
(a₃) the micronized compound of formula 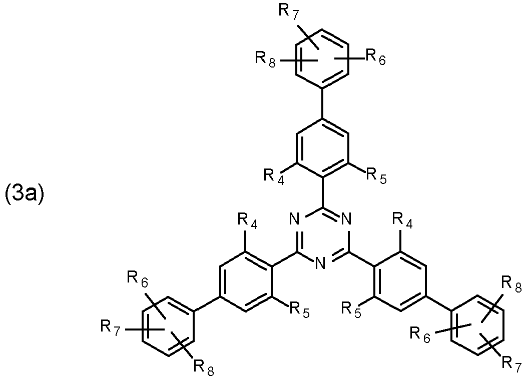 ; wherein
   R₄ and R₅ independently from each other are hydrogen; C₁-C₁₈alkyl; or C₆-C₁₂aryl;
   R₆, R₇ and R₈ independently from each other, are hydrogen; C₁-C₁₈alkyl; or a radical of formula 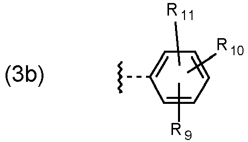 ; wherein
   R₉, R₁₀ and R₁₁ independently from each other, are hydrogen; or C₁-C₁₈alkyl; and
(a₄) the micronized compound of formula 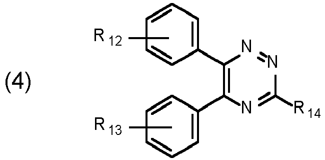 wherein
   R₁₂ and R₁₃, independently from each other, represent hydrogen; halogen; C₁-C₁₂alkyl: C₁-C₁₈hydroxy; C₁-C₁₈alkoxy; poly(ethoxy)-alkoxy with a C₁-C₄ alkyl fragment and an ethoxy number ranging from 1 to 4; C₁-C₄mono- or dialkyl amino,
   R₁₄ represents halogen; hydroxy; amino; phenyl which is optionally substituted 1 to 3 times by a hydroxy radical situated at least in a para or phenyl position, possibly 1 to 3 times substituted in an ortho, meta or para position by a C₁-C₁₂alkoxy or cyano or C₁-C₇alkylamino group;
   0.1 to 20 % by weight of at least one dispersant (c) selected from glutamates;
   0 to 1 % by weight of Ingredients based on polydimethylsiloxane/silica; and
   ad 100 % water.

Furthermore, an aqueous dispersion (A), comprising a micronized organic UV filter selected from
(a₁) the micronized compound of formula 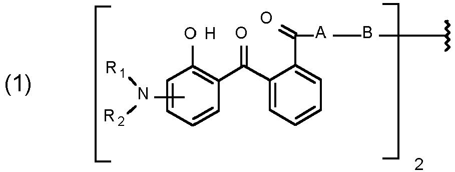 , wherein
   R₁ and R₂ independently from each other are C₁-C₂₀alkyl; C₂-C₂₀alkenyl; C₃-C₁₀cycloalkyl; C₃-C₁₀cycloalkenyl; or R₁ and R₂ together with the linking nitrogen atom form a 5- or 6-membered heterocyclic ring;
   A is --N(R₃)-; -O-; or the direct bond;
   B is a bivalent radical selected from alkylene, cycloalkylene alkenylene or phenylene radical which is optionally substituted by a carbonyl- or carboxy group; a radical of formula *-CH₂C≡C-CH₂-* ; or the divalent radical *-B-* corresponds to the formula 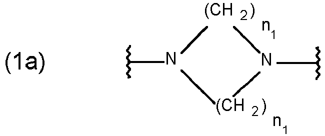 ; wherein
   n₁ is a number from 1 to 3;
      and
   R₃ is hydrogen; C₁-C₅alkyl; or hydroxy-C₁-C₅alkyl;
(az) the micronized compound of formula 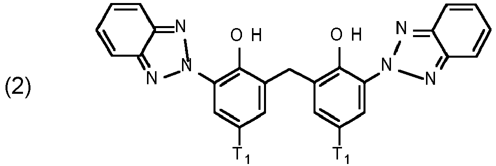 , wherein
   T₁ is hydrogen; C₁-C₁₂alkyl; preferably iso-octyl, or C₁-C₄alkyl substituted by phenyl
(a₃) the micronized compound of formula 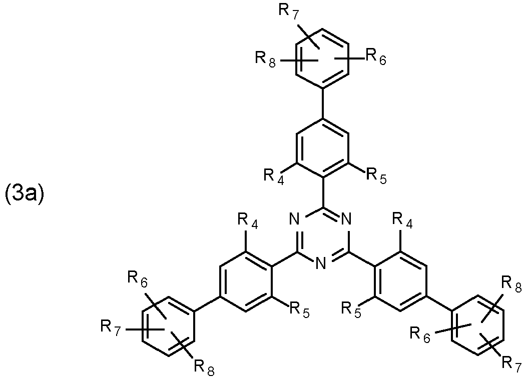 ; wherein
   R₄ and R₅ independently from each other are hydrogen; C₁-C₁₈alkyl; or C₆-C₁₂aryl;
   R₆, R₇ and R₈ independently from each other, are hydrogen; C₁-C₁₈alkyl; or a radical of formula 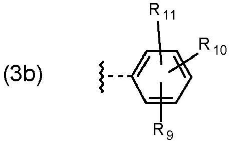 ; wherein
   R₉, R₁₀ and R₁₁ independently from each other, are hydrogen; or C₁-C₁₈alkyl; and
(a₄) the micronized compound of formula 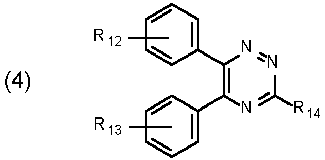 wherein
   R₁₂ and R₁₃, independently from each other, represent hydrogen; halogen; C₁-C₁₂alkyl: C₁-C₁₈hydroxy; C₁-C₁₈alkoxy; poly(ethoxy)-alkoxy with a C₁-C₄ alkyl fragment and an ethoxy number ranging from 1 to 4; C₁-C₄mono- or dialkyl amino,
   R₁₄ represents halogen; hydroxy; amino; phenyl which is optionally substituted 1 to 3 times by a hydroxy radical situated at least in a para or phenyl position, possibly 1 to 3 times substituted in an ortho, meta or para position by a C₁-C₁₂alkoxy or cyano or C₁-C₇alkylamino group; and
(b) a vinylpyrrolidone copolymer or homopolymer is disclosed.

C₁-C₂₀Alkyl denotes a linear or branched, unsubstituted or substituted alkyl group such as, for example, methyl, ethyl, propyl, isopropyl, n-butyl, n-hexyl, cyclohexyl, n-decyl, n-dodecyl, n-octadecyl eicosyl, methoxyethyl, ethoxypropyl, 2-ethylhexyl, hydroxyethyl, chloropropyl, N,N-diethylaminopropyl, cyanoethyl, phenethyl, benzyl, p-tert-butylphenethyl, p-tert-octylphe-noxyethyl, 3-(2,4-di-tert-amylphenoxy)-propyl, ethoxycarbonylmethyl-2-(2-hydroxyethoxy)-ethyl or 2-furylethyl.

C₂-C₂₀alkenyl is for example allyl, methallyl, isopropenyl, 2-butenyl, 3-butenyl, isobutenyl, n-penta-2,4-dienyl, 3-methyl-but-2-enyl, n-oct-2-enyl, n-dodec-2-enyl, iso-dodecenyl, n-dodec-2-enyl or n-octadec-4-enyl.

C₃-C₁₀cycloalkyl is for example cyclopropyl, cyclobutyl, cyclopentyl, cycloheptyl, cyclooctyl, cyclononyl or cyclodecyl and preferably cyclohexyl. These radicals may be substituted, for example by one or more or equal or different C₁-C₄alkyl radicals, preferably by methyl, and/or hydroxy. If cycloalkyl radicals are substituted by one or more radicals, they are preferably substituted by one, two or four, preferably by one or two equal or radicals.

C₃-C₁₀cycloalkenyl is for example cyclopropenyl, cyclobutenyl, cyclopentenyl, cycloheptenyl, cycloocentyl, cyclononenyl or cyclodecenyl and preferably cyclohexenyl. These radicals may be substituted with one or more equal or different C₁-C₄alkyl radical, preferably with methyl, and/or hydroxy. If cycloalkenyl radicals are substituted with one or more radicals they are preferably substituted with one, two, three or four, preferably with one or two equal or different radicals.

Hydroxy substituted C₁-C₅alkyl groups are for example hydroxymehtyl, hydroxyethyl, hydroxypropyl, hydroxybutyl or hydroxypentyl.

An alklyene radical is preferably a C₁-C₁₂alkylene radical, like for example methylene, ethylene, propylene, butylene, hexylene or octylene.

A cycloalklyene radical is preferably a cyclo-C₃-C₁₂alkylene radical, like for example cyclopropylene, cyclobutylene, cyclohexylene or cyclooctylene.

The alkylene- or cycloalkylene radicals may optionally be substituted by one or more C₁-Csalkyl radicals.

If R₁ and R₂ are heterocyclic radicals, these comprise one, two, three or four equal or different ring hetero atoms. Special preference is given to heterocycles which contain one, two or three, especially one or two, identical or different hetero atoms. The heterocycles may be mono- or poly-cyclic, for example mono-, bi- or tri-cyclic. They are preferably mono- or bicyclic, especially monocyclic. The rings preferably contain 5, 6 or 7 ring members. Examples of monocyclic and bicyclic heterocyclic systems from which radicals occurring in the compounds of formula (1) or (2) may be derived are, for example, pyrrole, furan, thiophene, imidazole, pyrazole, 1,2,3-triazole, 1,2,4-triazole, pyridine, pyridazine, pyrimidine, pyrazine, pyran, thiopyran, 1,4-dioxane, 1,2-oxazine, 1,3-oxazine, 1,4-oxazine, indole, benzothiophene, benzofuran, pyrrolidine, piperidine, piperazine, morpholine and thiomorpholine.

Preferably the dispersion (A) does not contain any thickeners, which are preferably selected from propylene glycol, carbomer and vegetable gums, most preferablyxanthan gum.

Preference is given to compounds of formula (1), wherein
R₁ and R₂ independently from each other are hydrogen; or C₁-C₂₀alkyl; or R₁ and R₂ together with the linking nitrogen atom form a 5- or 6-membered heterocyclic ring;
B is an alkylene-, radical which is optionally interrupted by a carbonyl- or carboxy group; or a radical of formula (1b);
A is -O-; or -N(R₃)-; or the direct bond and
R₃ is hydrogen; C₁-C₅alkyl; or hydroxy-C₁-C₅alkyl.

Of preferred interest are compounds of formula (1), wherein
R₁ and R₂ are C₁-C₂₀alkyl, preferably C₁-C₅alkyl; and most preferably ethyl.

Preferably R₁ and R₂ in formula (1) have the same definition.

Mostly preferred are compounds of formula (1), wherein
B corresponds to formula (1b).

Very most preferably, the micronized organic UV filter (a₁) corresponds to formula

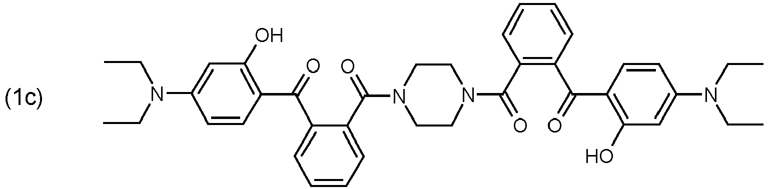

Preferably, the micronized organic UV filter (a₂) corresponds to formula

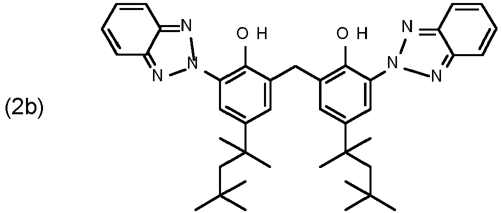

Preferably, the micronized organic UV filter (a₃) corresponds to formula

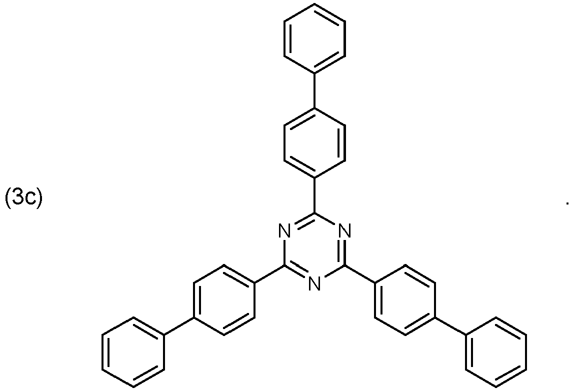

Preferably, the micronized organic UV filter (a₄) corresponds to formula

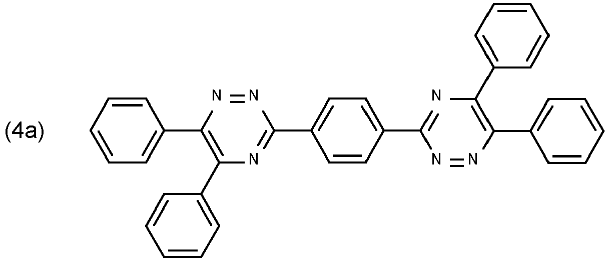

Vinylpyrrolidone copolymers or homopolymers according to component (b) refer to polyvinylpyrrolidone and water-soluble polymers which contain 1-vinyl-2-pyrrolidone monomers and are accepted in cosmetic formulations for leave-on skin application to effect the thickening of micronized UV filter dispersions.

Preferably polyvinylpyrrolidone or Poly[1-(2-oxo-1-pyrrolidinyl)ethylen] (PVP; CAS-Number: 9003-39-8) is used as component (b). PVP is the linear polymer that consists of 1-vinyl-2-pyrrolidone monomers conforming generally to the formula

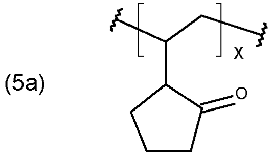

Preferably, the dispersion (A) comprises
the micronized organic UV filter (a₁) corresponding to formula 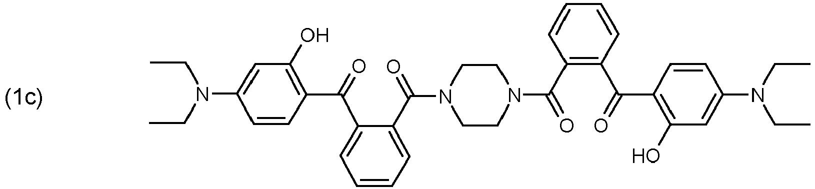 ; and
(b) polyvinylpyrrolidone of formula 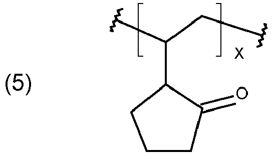
Preferably the dispersion (A) does not contain any glycol (diol) compounds.

A diol is a chemical compound containing two hydroxyl groups (-OH groups). This pairing of functional groups is pervasive and many subcategories have been identified. The most common industrial diol is ethylene glycol. Examples of diols in which the hydroxyl functional groups are more widely separated include 1,2-, 1,3-, 1,4-, 1-5 and longer diols, for example 1,4-butanediol HO-(CH₂)₄-OH, 1,5-pentanediol HO-(CH₂)₅-OH and bisphenol A, propylene-1,3-diol or beta propylene glycol, HO-CH₂-CH₂-CH₂-OH, 2-methyl-2-propyl-1,3-propanediol and neopentyl glycol.

More examples of glycol compounds are Arachidyl Glycol, Benzyl Glycol, Butoxydiglycol, Caprylyl Glycol, Ceteareth-60 Myristyl Glycol, Cetyl Glycol, C14-30 Glycol, Decylene Glycol, Diethylene Glycol, Dimethoxydiglycol, Dimethylol Glycol, Dipropylene Glycol, Ethoxydiglycol, Hexacosyl Glycol, Hexylene Glycol, Lauryl Glycol, Methoxydiglycol, Methylene Glycol, Myristyl Glycol, Octacosanyl Glycol, Pentylene Glycol, Poly(1,2-Butanediol)-6 Propylene Glycol, Polybutylene Glycol-10, Stearyl Glycol, Triethylene Glycol or Tripropylene Glycol.

The UV filters (a₁) - (a₄) which are used in the present invention are present in the micronized state and are preferably prepared by wet-milling processes. Any known process suitable for the preparation of microparticles can be used for the preparation of the micronized UV absorbers, for example:
- wet-milling (low-viscosity micronisation process for pumpable dispersions), with a hard grinding medium, for example zirconium silicate balls in a ball mill, and a protective surfactant or a protective polymer in water or in a suitable organic solvent;
- wet-kneading (high-viscosity micronisation process for non-pumpable pastes) using a continuous or discontinuous (batch) kneader. For a wet-kneading process, a solvent (water or cosmetically acceptable oils), a grinding aid (surfactant, emulsifier) and a polymeric grinding aid may be used.
- spray-drying from a suitable solvent, for example aqueous suspensions or suspensions containing organic solvents, or true solutions in water, ethanol, dichloroethane, toluene or N-methylpyrrolidone etc..
- by expansion according to the RESS process (Rapid Expansion of Supercritical Solutions) of supercritical fluids (e.g. CO₂) in which the UV filter or filters is/are dissolved, or the expansion of liquid carbon dioxide together with a solution of one or more UV filters in a suitable organic solvent;
- by reprecipitation from suitable solvents, including supercritical fluids (GASR process = Gas Anti-Solvent Recrystallisation / PCA process = Precipitation with Compressed Anti-solvents).

Wet-milling and wet-kneading are the preferably used processes.

As milling apparatus for the preparation of the micronized organic UV absorbers (a₁) - (a₄) there may be used, for example, a jet mill, ball mill, vibratory mill or hammer mill, preferably a high-speed mixing mill. Even more preferable mills are modern ball mills; manufacturers of these types of mill are, for example, Netzsch (LMZ mill), Drais (DCP-Viscoflow or Cosmo), Bühler AG (centrifugal mills) or Bachhofen. The grinding is preferably carried out with a grinding aid.

Examples of kneading apparatus for the preparation of the micronised organic UV absorbers (a₁) - (a₄) are typical sigma-blade batch kneaders but also serial batch kneaders (IKA-Werke) or continuous kneaders (Continua from Werner und Pfleiderer).

For each example of the aqueous dispersion, the manufacturing process is carried out as following:
The UV filters (a₁) - (a₄) respectively are added to the aqueous homogenous dispersion comprising the dispersing agent containing water. This slurry is then milled together with zirconium silicate beads (diameter 0.1 to 4 mm) as grinding medium in a ball mill (as described previously) to a mean particle size of d₅₀ from 100nm to 170nm. After the micropigment dispersion of the UV filters (a₁) - (a₄) respectively is obtained, it may be pH-adjusted and / or is formulated with a preservation system, and polyvinylpyrrolidone (PVP) (b) is added as a thickener system.

PVP is essentially water soluble and stock solutions with a high level of the polymer can be obtained. Thus the preparation of a pre-dispersion of the thickener in organic solvents, like propylene glycol, becomes obsolete.

The grinding of the sparingly soluble organic compounds used in the present invention is preferably carried out in the presence of a grinding aid.

Grinding aids may be any surface active ingredients that can be used as dispersing agents. Such surface active ingredients may comprise an anionic, a nonionic, an amphoteric or/and a cationic surfactant, or mixtures thereof.

Preferably the grinding aid is used in a concentration of 0.1 to 20% by weight, preferably 0.4 to 15 % b.w. based on the total weight of the UV protection composition.

Useful grinding aids are alkyl polyglucoside (ga₀).

Useful grinding aids are carboxylic acids and their salts (ga₁), for example
- organic basis soap such as linear C₆-C₂₄ fatty acids (capric/caprylic, myristic, palmitoleic, isostearic, linoleic, arachidic, behenic, erucic acids) or branched carboxylic acids or hydroxycarboxylic acids.

Further useful grinding aids are fatty acid esters (ga₂), for example
- esters of linear C₃-C₇ or C₂₃-C₂₄fatty alcohols with linear fatty acids having from 6 to 11 carbon atoms or more than 22 carbon atoms in the alkyl group.

Preferred is isocetyl isostearate or glycol oleate.

Further useful grinding aids (ga₃) are alkyl phosphates or phosphoric acid esters; such as DEA-oleth-3 phosphate.

Further useful grinding aids (ga₄) are ethoxylated carboxylic acids or polyethyleneglycol (PEG) esters such as PEG-n Acylates, except PEG-n Stearate, PEG-n Oleate, PEG-n Cocoate, in which the carboxylic acids have alkyl group, ethoxylated or not, with 8 to 22 carbon atoms such as butyric, caproic, caprylic, capric, lauric, myristic, myristoleic, palmitic, palmitoleic, linoleic, arachidic, arachidonic, behenic, eicosapentanoic, erucic or docosahexanoic.

Preferred is PEG-20 laurate.

Further useful grinding aids (ga₅) are fatty alcohol polyethyleneglycol (PEG) ethers of fatty alcohols.

Where linear or branched fatty alcohols having from 8 to 22 carbon atoms (isopropyl, ethylhexyl, capryl/caprylyl, isotridecyl, myristyl, palmoleyl, isostearyl, linoyl, linolenyl, arachidyl, behenyl or erucyl.

Preferred is ceteth-10, Laureth-7 or PEG-10 behenyl ether (Beheneth-10).

Further useful grinding aids (ga₇) are ethoxylated alkylphenols or ethoxylated alkylphenyl ethers such as PEG-10 nonyl phenyl ether.

Further useful grinding aids (ga₈) are esters of polyol and mono-, di- or tri-glycerides such as PEG-10 polyglyceryl-2 laurate.

Further useful grinding aids (ga₉) are esters of fatty acids and saccharose such as PEG-120 methyl glucose dioleate or polyglyceryl-3 methylglucose distearate.

Further useful grinding aids (ga₁₀) are sorbitan mono- and di-esters of saturated and/or unsaturated fatty acids such as PEG-20 sorbitan Isostearate and polysorbate-80.

Further useful grinding aids (ga₁₁) are surfactants which are generally acting as detergent or cleansing agents. Examples are listed below:

### A. Anionic surfactants

Anionic surfactants are designated as such due to the presence of a negatively charged fatty moiety. Such ionised moiety can be a carboxylate, a sulfate, a sulfonate or a phosphate.

General form of anionic surfactant is
R X⁻ M+, wherein
R defines the carbon chain length;
X: is negatively charged species such as carboxylate (RCOO⁻), sulfate (ROSO₂O⁻), sulfonate (RSO₂O⁻ ), or phosphate (ROPO(OH)O⁻ )
M is a neutralizing group such as sodium, ammonium, TEA or magnesium.

### a. Sulfates; Sulfuric acids and salts

Preferred is Sodium dicocoylethylene diamine PEG-15 sulfate.

### b. Sulfonates; Sulfonic acids and salts

Acyl Isethionates salts such as sodium acyllsethionate, sodium Cocoyl Isethionate, alkylaryl sulfonates salts such as sodium alkylbenzene sulfonate, sodium dodecylbenzene sulfonate; alkyl Sulfonates salts such as sodium alkylether sulfonate (sodium C12-15 pareth-15 sulfonate); Sodium C₁₄-C₁₆ olefin sulfonate, Sodium decylglucosides Hydroxypropyl sulfonate, or Sodium Laurylglucosides Hydroxypropyl sulfonate

Preferred is hydroxypropyl sulfonate.

### c. Sulfosuccinates; sulfosuccinic acids and salts

Preferred is disodium alkylamido PEG-n sulfosuccinate (Disodium oleamido MEA-sulfosuccinate).

### d. Phosphates; Phosphoric acids and salts

PEG-n alkyl phosphates such as DEA oleth-10 phosphate, di PEG-n alkyl phosphates (di-esters) such as dilaureth-4 phosphate.

### e. Acylamino acid and salts

Acyl glutamates such as Di-TEA palmitoyl aspartate, sodium hydrogenated tallow glutamate; Sodium stearoyl glutamate; Sodium Cocoyl Glutamate; Disodium Cocoyl Glutamate; acyl peptides such as palmitoyl hydrolysed milk protein, sodium cocoyl hydrolysed soy protein, TEA-cocoyl hydrolysed collagen; Sarcosinates or acyl sarcosides such as myristoyl sarcosine, Sodium lauroyl sarcosinate, sodium myristoyl sarcosinate TEA-lauroyl sarcosinate; taurates and sodium methyl acyltaurates such as sodium lauroyl taurate or sodium methyl cocoyl taurate.

### B. Non ionic surfactants

### Amine oxides

Examples are cocamidopropyl amine oxide or lauramine oxide.

### C. Amphoteric or Zwitterionic surfactants

Surfactants that carry a positive charge in strongly acidic media, carry a negative charge in strongly basic media and form zwitterionic species at intermediate pH.

### C1. Acyl/Dialkyl ethylenediamines

Examples are disodium acylamphodipropionate, sodium acylamphohydroxypropylsulfonate, disodium acylamphodiacetate, sodium acylamphopropionate.

### D. Cationic surfactants

Surfactants that carry a positive charge; major interesting hair care for conditioning and antistatic effects.

### D1. Alkylamines

Such as dimethyl alkylamine (dimethyl lauramine), dihydroxyethyl alkylamine dioleate, Acylamidopropyldimethylamine lactate (cocamidopropyl dimethylamine lactate).

### D2. Alkyl imidazolines

Such as alkyl hydroxyethyl imidazoline, Ethylhydroxymethyl oleyl oxazoline, alkyl aminoethyl imidazoline.

### D3. Quaternary compounds

Examples are dialkyldimonium chloride (hydroxyethyl cetyldimonium chloride), alkylamidopropyl alkyldimonium tosylate (Cocamidopropyl ethyldimonium ethosulfate.

Further useful grinding aids (ga₁₂) are silicones or organosubstituted polysiloxanes, i.e. any organosilicon polymers or oligomers having a linear or cyclic, branched or crosslinked structure, of variable molecular weight, and essentially based of recurring structural units in which the silicone atoms are linked to each other by oxygen atoms (siloxane bond SiOSi), optionally substituted hydrocarbon radicals being directly linked via a carbon atom to the silicone atoms.

### E. Silicium containing compounds

### E1. Silanol compounds or dimethiconols

Dimethyl siloxane terminated with hydroxyl groups (-OH) of the general formula

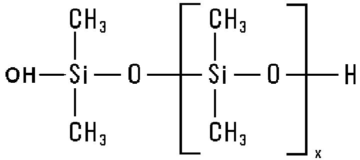

### E2. Silicone elastomers & resins

Crosslinking of siloxane structures such as Dimethicones.
Elastomer: medium crosslinking with a density that allows elongation/distorsion of the molecule. PEG-modified Dimethicone Crosspolymers are excluded.
Resin: high crosslinking with a density that provides some rigidity to the molecule

### E3. Silicone elastomers as co-emulsifier systems

Dimethicone Crosspolymer in Cyclopentasiloxane; DC 9045 silicone elastomer blend (Dow Corning); Dimethicone Crosspolymer in Dimethicone; DC 9041 silicone elastomer blend (Dow Corning); polymer of Dimethicone (q.v.) crosslinked with a C3 to C20 alkyl group Dimethicone/ Vinyldimethicone Crosspolymer; DC 9506 powder (Dow Corning); Dimethicone/ Vinyldimethicone Crosspolymer in Cyclopentasiloxane; SFE 839 (GE silicones) or KSG 15(Shin-Etsu); copolymer of dimethylpolysiloxane crosslinked with vinyl dimethylpolysiloxane.

### E4. Resin silicones

Examples are dispersing agents such as KP-545 (Shin-Etsu); acrylates/dimethicone copolymer in cyclopentasiloxane; copolymer of dimethicone and one or more monomers of acrylic acid, methacrylic acid or one of their simple esters; Siloxysilicates such as Trimethylsiloxy-silicates ; T-resins; branched polymer of T-Units; Q-resins; branched polymer of Q-Units:

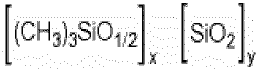

Film-forming and water-resistant agents such as Trimethylsiloxysilicate; SR 399 (GE Silicones) or Wacker-Belsil TMS803 (Wacker Chemie); mixtures from Dow Corning such as DC 749 cosmetic fluid (Trimethylsiloxysilicate in Cyclopentasiloxane) or DC 593 fluid (Trimethylsiloxysilicate in Dimethicone); Alkyl-Modified Siloxanes (AMS); AMS improve spreadability and wash-off resistance.

For inorganic sunscreens, it improves particle dispersion, reduce the re-agglomeration and better long-lasting effect on skin.

Alkyl Dimethicone of the general formula 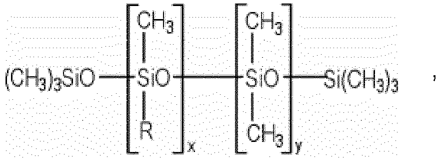 wherein
R is -(CH2)ₙ-CH₃.

For example: Bis-Phenylpropyl Dimethicone (SF 1555 fluid; GE Silicone).

Alkyl Methicone of the general formula 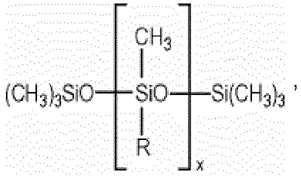 wherein
R is -(CH2)ₙ-CH₃.^

Silicone waxes such as DC 2503 cosmetic wax (Dow Corning); Stearyl Dimethicone; DC 2502 fluid (Dow Corning); Cetyl Dimethicone; DC AMS-C30 wax (Dow Corning); C30-C45 Alkyl Methicone; DC 580 wax (Dow Corning); Stearoxytrimethylsilane and Stearyl Alcohol.

Also suitable are simethicones, which are mixtures of dimethicones having an average chain length of from 200 to 300 dimethylsiloxane units with hydrogenated silicates. A detailed survey by Todd et al. of suitable volatile silicones may in addition be found in Cosm. Toil. 91, 27 (1976).

Silicone emulsifiers particularly suitable for such kind of emulsions are those corresponding to the following formula 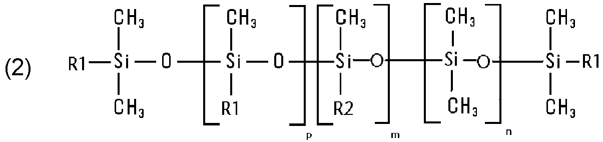 wherein
m is a number from 1 to 20;
n is a umber from 0 to 500; and
p is a number from 0 to 50;
R₁ is linear or branched C₁- C₃₀ alkyl radical or phenyl radical;
R₂ is -C_{c}H_{2c}(-O- C₂H₄)ₐ-(-O-C₃H₆)_{b}-(-O-C₄H₈)_{d}-R_{3;}
R₃ is H, -OH; linear or branched C₁- C₁₂alkyl, linear or branched C₁- C₆alkoxy, or linear or branched C₂-C₁₂acyloxy; -NHCH₂CH₂COOM; aminoalkyl radical optionally substituted on the amine function; -NHCO(CH₂)_{d}-COOM, C₁-C₃₀carboxyacyl radical;
M is H; Na; K; Li; NH₄; or organic amine; optionally substituted phosphono group; -NHCO(CH₂)_{d} OH; NH₃Y;
Y is a monovalent organic or inorganic anion such as Cl, Br, Sulfate, Carboxylate (Acetate, Lactate, Citrate);
a is a number from 0 to 100;
c is a number from 0 to 5;
b is a number from 0 to 50; and
d is a number from 0 to 10.

These compounds represent the oxyalkylenated organo-modified silicones. Other nomenclature used is PEG/PPG Dimethicones (Dimethicone copolyols) or Silicone polyethers which clearly show surface active properties necessary to emulsify.

Silicone emulsifiers which are particularly recommended correspond to formula 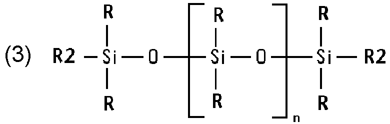 wherein
R is linear or branched C₁-C₃₀ alkyl radical or phenyl radical;
R₂ is -C_{c}H2_{c}-(-O-C₂H₄)ₐ-(-O-C₃H₆)_{b}- O(-C₄H₈)_{d} -R₃;
n is a number from1 to 500; and
R₃, a, b, c and d have the same meaning as described above

Preferred is dimethicone PEG/PPG - 7/4 phosphate.

Preferably, the micronized insoluble organic UV absorber, prepared according to the method of the present invention, has a mean particle size in the range of from 0.01 to 2, more preferably from 0.02 to 1.5, especially from 0.05 to 1.0µ.

In a further embodiment of the present invention the dispersion of micronized UV filters is created in such a way that no thickener is needed to adjust the required viscosity level. These dispersions show a thixotropic behaviour, meaning that they are more fluid at high shear of the liquid, while at low shear the viscosity increases. This slows down the sedimentation of the dispersed UV filter particles when the product is stored, while at the same time the dispersion is pumpable when taken out of the container, or is formulated into the consumer product.

It was found that several emulsifiers are capable to act as grinding aids in the micronization process and stabilize the final micronized dispersion. Specifically, the grinding aids are selected from the group of glutamates. These materials are wide-spread in the formulation of sunscreens, daily creams and other leave-on cosmetics.

Thus, another aspect of the present invention relates to an aqueous dispersion (B) comprising
20 to 60 % by weight of at least one UV filter selected from
(a₁) the micronized compound of formula 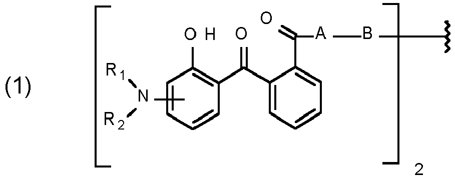 , wherein
   R₁ and R₂ independently from each other are C₁-C₂₀alkyl; C₂-C₂₀alkenyl; C₃-C₁₀cycloalkyl; C₃-C₁₀cycloalkenyl; or R₁ and R₂ together with the linking nitrogen atom form a 5- or 6-membered heterocyclic ring;
   A is --N(R₃)-; -O-; or the direct bond;
   B is a bivalent radical selected from alkylene, cycloalkylene alkenylene or phenylene radical which is optionally substituted by a carbonyl- or carboxy group; a radical of formula *-CH₂C≡C-CH₂-* ; or the divalent radical *-B-* corresponds to the formula 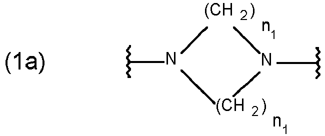 ; wherein
   n₁ is a number from 1 to 3;
   A is --N(R₃)-; or -O-; and
   R₃ is hydrogen; C₁-C₅alkyl; or hydroxy-C₁-C₅alkyl;
(az) the micronized compound of formula 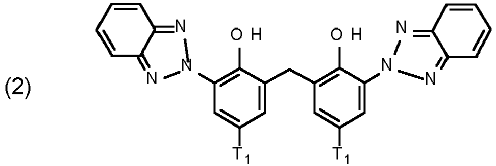 , wherein
   T₁ is hydrogen; C₁-C₁₂alkyl; preferably iso-octyl, or C₁-C₄alkyl substituted by phenyl;
(a₃) the micronized compound of formula 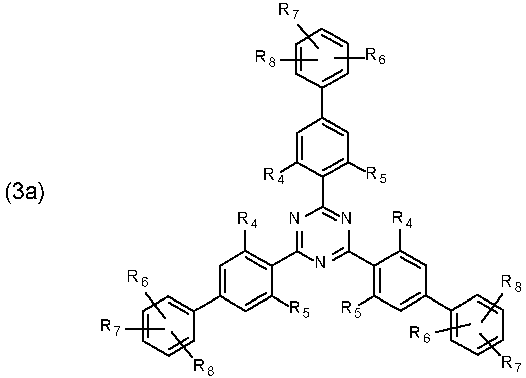 ; wherein
   R₄ and R₅ independently from each other are hydrogen; C₁-C₁₈alkyl; or C₆-C₁₂aryl;
   R₆, R₇ and R₈ independently from each other, are hydrogen; C₁-C₁₈alkyl; or a radical of formula 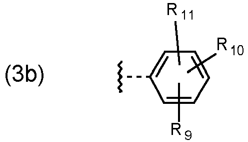 ; wherein
   R₉, R₁₀ and R₁₁ independently from each other, are hydrogen; or C₁-C₁₈alkyl; and
(a₄) the micronized compound of formula 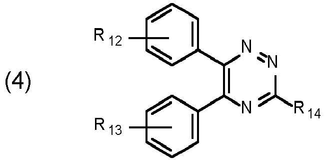 wherein
   R₁₂ and R₁₃, independently from each other, represent hydrogen; halogen; C₁-C₁₂alkyl: C₁-C₁₈hydroxy; C₁-C₁₈alkoxy; poly(ethoxy)-alkoxy with a C₁-C₄ alkyl fragment and an ethoxy number ranging from 1 to 4; C₁-C₄mono- or dialkyl amino,
   R₁₄ represents halogen; hydroxy; amino; phenyl which is optionally substituted 1 to 3 times by a hydroxy radical situated at least in a para or phenyl position, possibly 1 to 3 times substituted in an ortho, meta or para position by a C₁-C₁₂alkoxy or cyano or C₁-C₇alkylamino group;
   0.1 to 20 % by weight of at least one dispersant (c) selected from glutamates;
   0 to 1 % by weight of Ingredients based on polydimethylsiloxane/silica; and
   ad 100 % water.

This dispersion formulation enables the preparation of personal care products which contain at least one of the UV filters (a₁) - (a₄) and "leave-on" formulation additives only.

Preferably, the aqueous dispersion (A) disclosed herein comprises
20 to 60 % by weight of at least one micronized UV filter selected from
(a₁) the micronized compound of formula 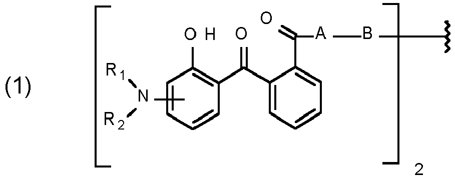 , wherein
   R₁ and R₂ independently from each other are C₁-C₂₀alkyl; C₂-C₂₀alkenyl; C₃-C₁₀cycloalkyl; C₃-C₁₀cycloalkenyl; or R₁ and R₂ together with the linking nitrogen atom form a 5- or 6-membered heterocyclic ring;
   A is --N(R₃)-; -O-; or the direct bond;
   B is a bivalent radical selected from alkylene, cycloalkylene alkenylene or phenylene radical which is optionally substituted by a carbonyl- or carboxy group; a radical of formula 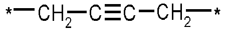 ; or the divalent radical *-B-* corresponds to the formula 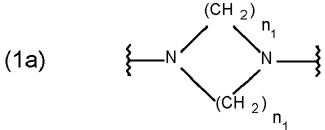 ; wherein
   n₁ is a number from 1 to 3;
   A is --N(R₃)-; or -O-; and
   R₃ is hydrogen; C₁-C₅alkyl; or hydroxy-C₁-C₅alkyl;
(az) the micronized compound of formula 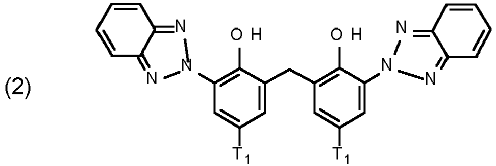 , wherein
   T₁ is hydrogen; C₁-C₁₂alkyl; preferably iso-octyl, or C₁-C₄alkyl substituted by phenyl;
(a₃) the micronized compound of formula 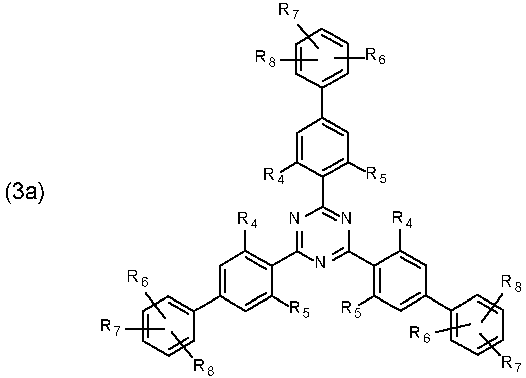 ; wherein
   R₄ and R₅ independently from each other are hydrogen; C₁-C₁₈alkyl; or C₆-C₁₂aryl;
   R₆, R₇ and R₈ independently from each other, are hydrogen; C₁-C₁₈alkyl; or a radical of formula 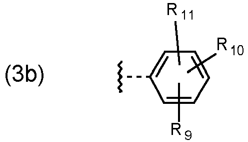 ; wherein
   R₉, R₁₀ and R₁₁ independently from each other, are hydrogen; or C₁-C₁₈alkyl; and
(a₄) the micronized compound of formula 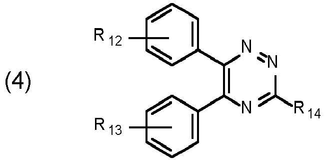 wherein
   R₁₂ and R₁₃, independently from each other, represent hydrogen; halogen; C₁-C₁₂alkyl: C₁-C₁₈hydroxy; C₁-C₁₈alkoxy; poly(ethoxy)-alkoxy with a C₁-C₄ alkyl fragment and an ethoxy number ranging from 1 to 4; C₁-C₄mono- or dialkyl amino,
   R₁₄ represents halogen; hydroxy; amino; phenyl which is optionally substituted 1 to 3 times by a hydroxy radical situated at least in a para or phenyl position, possibly 1 to 3 times substituted in an ortho, meta or para position by a C₁-C₁₂alkoxy or cyano or C₁-C₇alkylamino group;
   0.1 to 20 % by weight of at least one dispersant;
   0.1 to 2 % by weight of a vinylpyrrolidone copolymer or homopolymer (b);
   0 to 1 % by weight of Ingredients based on polydimethylsiloxane/silica; and
   ad 100 % water.

In another embodiment according to the present invention the aqueous dispersion (B) comprises
20 to 60 % by weight of at least one micronized UV filter selected from
(a₁) the micronized compound of formula 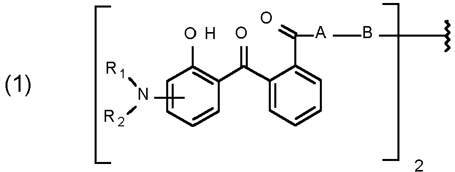 , wherein
   R₁ and R₂ independently from each other are C₁-C₂₀alkyl; C₂-C₂₀alkenyl; C₃-C₁₀cycloalkyl; C₃-C₁₀cycloalkenyl; or R₁ and R₂ together with the linking nitrogen atom form a 5- or 6-membered heterocyclic ring;
   A is --N(R₃)-; -O-; or the direct bond;
   B is a bivalent radical selected from alkylene, cycloalkylene alkenylene or phenylene radical which is optionally substituted by a carbonyl- or carboxy group; a radical of formula *-CH₂C≡C-CH₂-* ; or the divalent radical *-B-* corresponds to the formula 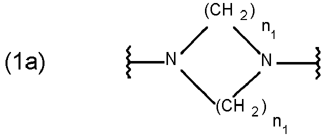 ; wherein
   n₁ is a number from 1 to 3;
   A is --N(R₃)-; or -O-; and
   R₃ is hydrogen; C₁-C₅alkyl; or hydroxy-C₁-C₅alkyl;
(az) the micronized compound of formula 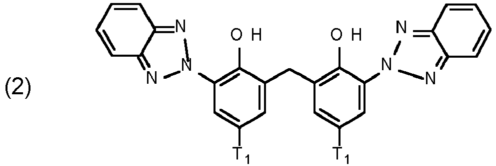 , wherein T₁ is hydrogen; C₁-C₁₂alkyl; preferably iso-octyl, or C₁-C₄alkyl substituted by phenyl;
(a₃) the micronized compound of formula 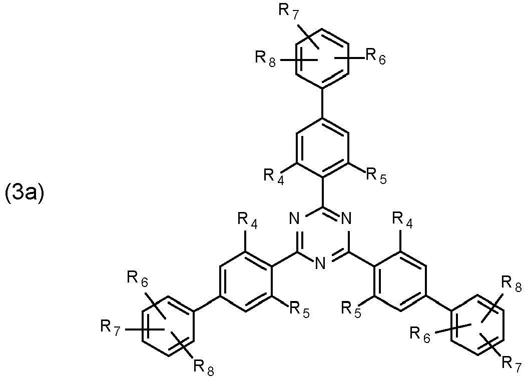 ; wherein
   R₄ and R₅ independently from each other are hydrogen; C₁-C₁₈alkyl; or C₆-C₁₂aryl;
   R₆, R₇ and R₈ independently from each other, are hydrogen; C₁-C₁₈alkyl; or a radical of formula 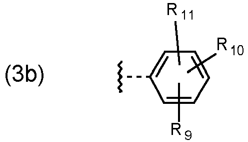 ; wherein
   R₉, R₁₀ and R₁₁ independently from each other, are hydrogen; or C₁-C₁₈alkyl; and
(a₄) the micronized compound of formula 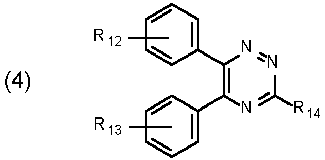 wherein
   R₁₂ and R₁₃, independently from each other, represent hydrogen; halogen; C₁-C₁₂alkyl: C₁-C₁₈hydroxy; C₁-C₁₈alkoxy; poly(ethoxy)-alkoxy with a C₁-C₄ alkyl fragment and an ethoxy number ranging from 1 to 4; C₁-C₄mono- or dialkyl amino,
   R₁₄ represents halogen; hydroxy; amino; phenyl which is optionally substituted 1 to 3 times by a hydroxy radical situated at least in a para or phenyl position, possibly 1 to 3 times substituted in an ortho, meta or para position by a C₁-C₁₂alkoxy or cyano or C₁-C₇alkylamino group;
   0.1 to 20 % by weight of at least one dispersant (c) selected from glutamates;
   0 to 1 % by weight of Ingredients based on polydimethylsioxane/silica; and
   ad 100 % water.

Also described herein, the aqueous dispersion (C) comprises
20 to 60 % by weight of at least one micronized UV filter selected from
(a₁) the micronized compound of formula 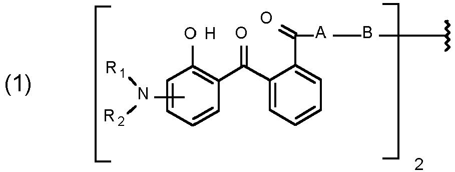 , wherein
   R₁ and R₂ independently from each other are C₁-C₂₀alkyl; C₂-C₂₀alkenyl; C₃-C₁₀cycloalkyl; C₃-C₁₀cycloalkenyl; or R₁ and R₂ together with the linking nitrogen atom form a 5- or 6-membered heterocyclic ring;
   A is --N(R₃)-; -O-; or the direct bond;
   B is a bivalent radical selected from alkylene, cycloalkylene alkenylene or phenylene radical which is optionally substituted by a carbonyl- or carboxy group; a radical of formula 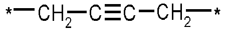 ; or the divalent radical *-B-* corresponds to the formula 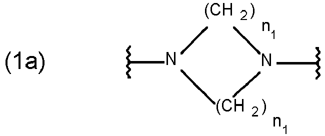 ; wherein
   n₁ is a number from 1 to 3;
   A is --N(R₃)-; or -O-; and
   R₃ is hydrogen; C₁-C₅alkyl; or hydroxy-C₁-C₅alkyl;
(az) the micronized compound of formula 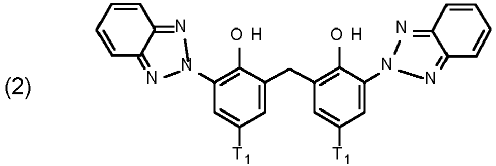 , wherein
   T₁ is hydrogen; C₁-C₁₂alkyl; preferably iso-octyl, or C₁-C₄alkyl substituted by phenyl;
(a₃) the micronized compound of formula 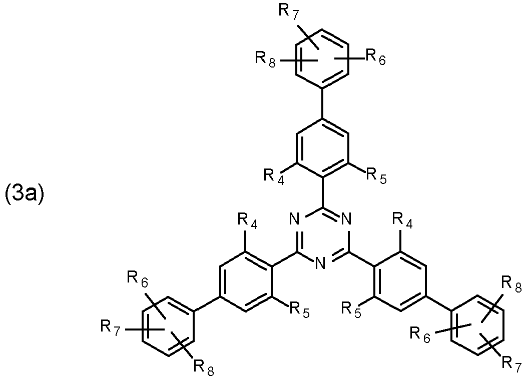 ; wherein
   R₄ and R₅ independently from each other are hydrogen; C₁-C₁₈alkyl; or C₆-C₁₂aryl;
   R₆, R₇ and R₈ independently from each other, are hydrogen; C₁-C₁₈alkyl; or a radical of formula 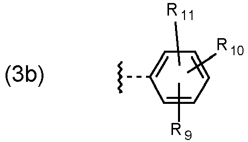 ; wherein
   R₉, R₁₀ and R₁₁ independently from each other, are hydrogen; or C₁-C₁₈alkyl; and
(a₄) the micronized compound of formula 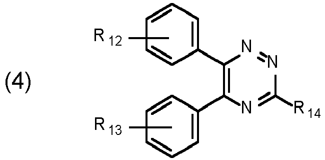 wherein
   R₁₂ and R₁₃, independently from each other, represent hydrogen; halogen; C₁-C₁₂alkyl: C₁-C₁₈hydroxy; C₁-C₁₈alkoxy; poly(ethoxy)-alkoxy with a C₁-C₄ alkyl fragment and an ethoxy number ranging from 1 to 4; C₁-C₄mono- or dialkyl amino,
   R₁₄ represents halogen; hydroxy; amino; phenyl which is optionally substituted 1 to 3 times by a hydroxy radical situated at least in a para or phenyl position, possibly 1 to 3 times substituted in an ortho, meta or para position by a C₁-C₁₂alkoxy or cyano or C₁-C₇alkylamino group;
   0.1 to 20 % by weight of at least one dispersant;
   0 to 1 % by weight of Ingredients based on polydimethylsioxane/silica (defoamer); and
   ad 100 % water.

The aqueous dispersions (A), (B) and (C) preferably do not contain any additional thickeners, which are preferably selected from propylene glycol, carbomer and vegetable gums, most preferablyxanthan gum.

The aqueous dispersions (A), (B) and (C) are particularly suitable for use in cosmetic and dermatological and pharmaceutical preparations.

Accordingly, the present invention also provides a sunscreen composition comprising
(i) 0.1 to 50%, preferably 0.5 to 20% by weight, based on the sunscreen composition of the aqueous dispersion (B) as defined above; and optionally
(ii) a dermatologically and pharmaceutically acceptable carrier.

The sunscreen composition of the present invention may be produced by physically blending the micronized formulation of an insoluble organic UV absorber and carrier components by any conventional method, e.g. by simply stirring the two materials together. In a preferred procedure, a mixture of the coarse, insoluble organic UV absorber, the grinding aid, and the milling bodies are ground until the coarse, insoluble organic UV absorber has been converted into micronized form, as described earlier in relation to the production of the micronized insoluble organic UV absorber. After filtering off the milling bodies, e.g. quartz sand, glass balls or zirconium silicate balls, the filtrate, consisting of the micronized insoluble organic UV absorber and grinding aid components, may be blended with a cosmetically compatible carrier.

The sunscreen composition of the present invention may be formulated as a water-in oil or an oil-in-water dispersion, an oil or oil-alcohol lotion, a vesicular dispersion of an ionic or nonionic amphiphilic lipid, a gel, a solid stick or an aerosol formulation.

When formulated as a water-in oil or an oil-in-water dispersion, the optional cosmetically acceptable carrier preferably comprises 5 to 50% of an oil phase, 5 to 20% of an emulsifier and 30 to 90% of water, each by weight based on the total weight of the carrier. The oil phase may comprise any oil conventionally used in cosmetic formulations, e.g., one or more of a hydrocarbon oil, a wax, a natural oil, a silicone oil, a fatty acid ester or a fatty alcohol. Preferred mono- or polyols are ethanol, isopropanol, propylene glycol, hexylene glycol, glycerin and sorbitol. The emulsifier also may comprise any emulsifier conventionally used in cosmetic formulations, e.g., one or more of an ethoxylated ester of a natural oil derivative such as a polyethoxylated ester of hydrogenated castor oil; a silicone oil emulsifier such as a silicone polyol; an optionally ethoxylated fatty acid soap; an ethoxylated fatty alcohol; an optionally ethoxylated sorbitan ester; an ethoxylated fatty acid; or an ethoxylated glyceride.

The sunscreen composition of the invention may also comprise further components which are known to perform a useful function in a sunscreen composition. Examples of such further components include, e.g., emollients, skin moisturizers, skin tanning accelerators, antioxidants, emulsion stabilizers, thickening agents such as xanthan, moisture-retention agents such as glycerin, film formers, preservatives, perfumes and colorants.

The UV absorber composition according to the present invention may comprise one or more than one additional UV absorbers as described in the Tables 1 and 2.

| Table 1: Suitable UV filter substances which can be additionally used with the UV absorbers according to the present invention | | |
|---|---|---|
| p-aminobenzoic acid derivatives, for example 4-dimethylaminobenzoic acid 2-ethylhexyl ester | | |
| salicylic acid derivatives, for example salicylic acid 2-ethylhexyl ester | | |
| benzophenone derivatives, for example 2-hydroxy-4-methoxybenzophenone and its 5-sulfonic acid derivative | | |
| diphenylacrylates, for example 2-ethylhexyl 2-cyano-3,3-diphenylacrylate, and 3-(benzofuranyl) 2-cyanoacrylate | | |
| 3-imidazol-4-ylacrylic acid and esters | | |
| benzofuran derivatives, especially 2-(p-aminophenyl)benzofuran derivatives, described in EP-A-582 189, US-A-5 338 539, US-A-5 518 713 and EP-A-613 893; | | |
| polymeric UV absorbers, for example the benzylidene malonate derivatives described in EP-A-709 080 | | |
| cinnamic acid derivatives, for example the 4-methoxycinnamic acid 2-ethylhexyl ester and isoamyl ester or cinnamic acid derivatives described in US-A-5 601 811 and WO 97/00851; | | |
| camphor derivatives, for example 3-(4'-methyl)benzylidene-bornan-2-one, 3-benzylidene-bornan-2-one, N-[2(and 4)-2-oxyborn-3-ylidene-methyl)-benzyl]acrylamide polymer, 3-(4'-trimethylammonium)-benzylidene-bornan-2-one methyl sulfate, 3,3'-(1,4-phenylene-dimethine)-bis(7,7-dimethyl-2-oxo-bicyclo[2.2.1]heptane-1-methanesulfonic acid) and salts, 3-(4'-sulfo)benzylidene-bornan-2-one and salts; camphorbenzalkonium methosulfate; | | |
| hydroxyphenyltriazine compounds, for example 2-(4'-methoxyphenyl)-4,6-bis(2'-hydroxy-4'-n-octyloxyphenyl)-1 ,3,5-triazine; 2,4-bis{[4-(3-(2-propyloxy)-2-hydroxy-propyloxy)-2-hydroxy]-phenyl}-6-(4-methoxyphenyl)-1,3,5-triazine; 2,4-bis{[4-(2-ethyl-hexyloxy)-2-hydroxy]-phenyl}-6-[4-(2-methoxyethyl-carboxyl)-phenylamino]-1,3,5-triazine; 2,4-bis{[4-(tris(trimethylsilyloxy-silylpropyloxy)-2-hydroxy]-phenyl}-6-(4-methoxyphenyl)-1,3,5-triazine; 2,4-bis{[4-(2"-methylpropenyloxy)-2-hydroxy]-phenyl}-6-(4-methoxyphenyl)-1,3,5-triazine; 2,4-bis{[4-(1',1',1',3',5',5',5'-heptamethyltrisilyl-2"-methyl-propyloxy)-2-hydroxy]-phenyl}-6-(4-methoxyphenyl)-1,3,5-triazine;2,4-bis{[4-(3-(2-propyloxy)-2-hydroxy-propyloxy)-2-hydroxy]-phenyl}-6-[4-ethylcarboxy)-phenylamino]-1,3,5-triazine; | | |
| physical sunscreens, coated or not coated, such as titanium dioxide, zinc oxide, iron oxides, mica, MnO, Fe₂O₃, Ce₂O₃, Al₂O₃, ZrO₂ (surface coatings: polymethylmethacrylate, methicone (methylhydrogenpolysiloxane as described in CAS 9004-73-3), dimethicone, isopropyl titanium triisostearate (as described in CAS 61417-49-0), metal soaps such as magnesium stearate (as described in CAS 4086-70-8), perfluoroalcohol phosphate such as C₉-C₁₅ fluoroalcohol phosphate (as described in CAS 74499-44-8; JP 5-86984; JP 4-330007)). The primary particle size is, on average, 15 nm - 35 nm and the particle size distribution is in the range 100 nm - 300 nm. | | |
| aminohydroxy-benzophenone derivatives disclosed in DE 100 11 317, EP 1 133 980 and EP 1 046 391 | | |
| phenyl-benzimidazole derivatives as disclosed in EP 1 167 358 | | |
| | | |

| Table 2: Suitable UV filter substances and adjuvants which can be additionally used with the UV absorbers according to the present invention | | |
|---|---|---|
| No. | Chemical Name | CAS No. |
| 1 | (+/-)-1,7,7-trimethyl-3-[(4-methylphenyl)methylene]bicyclo-[2.2.1]heptan-2-one; p-methyl benzylidene camphor | 36861-47-9 |
| 2 | 1,7,7-trimethyl-3-(phenylmethylene)bicyclo[2.2.1]heptan-2-one; benzylidene camphor | 15087-24-8 |
| 3 | (2-Hydroxy-4-methoxyphenyl)(4-methylphenyl)methanone | 1641-17-4 |
| 4 | 2,4-dihydroxybenzophenone | 131-56-6 |
| 5 | 2,2',4,4'-tetrahydroxybenzophenone | 131-55-5 |
| 6 | 2-Hydroxy-4-methoxy benzophenone; | 131-57-7 |
| 7 | 2-Hydroxy-4-methoxy benzophenone-5-sulfonic acid | 4065-45-6 |
| 8 | 2,2'-dihydroxy-4,4'-dimethoxybenzophenone | 131-54-4 |
| 9 | 2,2'-Dihydroxy-4-methoxybenzophenone | 131-53-3 |
| 10 | Alpha-(2-oxoborn-3-ylidene)toluene-4-sulphonic acid and its salts | 56039-58-8 |
| 11 | 1-[4-(1,1-dimethylethyl)phenyl]-3-(4-methoxyphenyl)propane-1,3-dione | 70356-09-1 |
| 12 | Methyl N,N,N-trimethyl-4-[(4,7,7-trimethyl-3-oxobicyclo[2,2,1]hept-2-ylidene)methyl]anilinium sulphate | 52793-97-2 |
| 22 | 3,3,5-Trimethyl cyclohexyl-2-hydroxy benzoate; homosalate | 118-56-9 |
| 23 | Isopentyl p-methoxycinnamate; isoamyl methoxy cinnamate | 71617-10-2 |
| 27 | Menthyl-o-aminobenzoate | 134-09-8 |
| 28 | Menthyl salicylate | 89-46-3 |
| 29 | 2-Ethylhexyl 2-cyano,3,3-diphenylacrylate; octocrylene | 6197-30-4 |
| 30 | 2-ethylhexyl 4-(dimethylamino)benzoate | 21245-02-3 |
| 31 | 2-ethylhexyl 4-methoxycinnamate; octyl methoxy cinnamate | 5466-77-3 |
| 32 | 2-ethylhexyl salicylate | 118-60-5 |
| 33 | Benzoic acid, 4,4',4"-(1,3,5-triazine-2,4,6-triyltriimino)tris(2-ethylhexyl)ester; 2,4,6-Trianilino-(p-carbo-2'-ethylhexyl-1'-oxi)-1,3,5-triazine | 88122-99-0 |
| 34 | 4-aminobenzoic acid | 150-13-0 |
| 35 | Benzoic acid, 4-amino-, ethyl ester, polymer with oxirane | 113010-52-9 |
| 38 | 2-phenyl-1H-benzimidazole-5-sulphonic acid; phenylbenzimidazolsulfonic acid | 27503-81-7 |
| 39 | 2-Propenamide, N-[[4-[(4,7,7-trimethyl-3-oxobicyclo[2.2.1]hept-2-ylidene)methyl]phenyl]methyl]-, homopolymer | 147897-12-9 |
| 40 | Triethanolamine salicylate | 2174-16-5 |
| 41 | 3,3'-(1,4-phenylenedimethylene)bis[7,7-dimethyl-2-oxo-bicyclo-[2.2.1]heptane-1 methanesulfonic acid]; Cibafast H | 90457-82-2 |
| 42 | Titanium dioxide | 13463-67-7 |
| 44 | Zinc oxide | 1314-13-2 |
| 46 | 1H-Benzimidazole-4,6-disulfonic acid, 2,2'-(1,4-phenylene)bis-, disodium salt | 180898-37-7 |
| 47 | Benzoic acid, 4,4'-[[6-[[4-[[(1,1-dimethylethyl)amino]carbonyl]-phenyl]amino]1,3,5-triazine-2,4-diyl]diimino]bis-, bis(2-ethylhexyl)ester; diethylhexyl butamido triazone; Uvasorb HEB | 154702-15-5 |
| 48 | Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane; Mexoryl XL | 155633-54-8 |
| 49 | Dimethicodiethylbenzalmalonate; Polysilicone 15; Parsol SLX | 207574-74-1 |
| 50 | Benzenesulfonic acid, 3-(2H-benzotriazol-2-yl)-4-hydroxy-5-(1-methylpropyl)-, monosodium salt; Tinogard HS | 92484-48-5 |
| 51 | Benzoic acid, 2-[4-(diethylamino)-2-hydroxybenzoyl]-, hexyl ester; Uvinul^{®} A Plus | 302776-68-7 |
| 52 | 1-Dodecanaminium, N-[3-[[4-(dimethylamino)benzoyl]amino]propyl]-N,N-dimethyl-, salt with 4-methylbenzenesulfonic acid (1:1); Escalol HP610 | 156679-41-3 |
| 53 | 1-Propanaminium, N,N,N-trimethyl-3-[(1-oxo-3-phenyl-2-propenyl)-amino]-, chloride | 177190-98-6 |
| 54 | 1H-Benzimidazole-4,6-disulfonic acid, 2,2'-(1,4-phenylene)bis- | 170864-82-1 |
| 55 | 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt) | 340964-15-0 |
| 56 | 2-Propenoic acid, 3-(1H-imidazol-4-yl)- | 104-98-3 |
| 57 | Benzoic acid, 2-hydroxy-, [4-(1-methylethyl)phenyl]methyl ester | 94134-93-7 |
| 58 | 1,2,3-Propanetriol, 1-(4-aminobenzoate); glyceryl PABA | 136-44-7 |
| 59 | Benzeneacetic acid, 3,4-dimethoxy-a-oxo- | 4732-70-1 |
| 60 | 2-Propenoic acid, 2-cyano-3,3-diphenyl-, ethyl ester | 5232-99-5 |
| 61 | Anthralinic acid, p-menth-3-yl ester | 134-09-8 |
| 62 | 2,2'-bis(1,4-phenylene)-1H-benzimidazole-4,6-disulphonic acid mono sodium salt or Disodium phenyl dibenzimidazole tetrasulfonate or Neoheliopan AP | 349580-12-7, |
| 63 | 1,3,5-Triazine-2,4,6-triamine, N,N'-bis[4-[5-(1,1-dimethylpropyl)-2-benzoxazolyl]phenyl]-N"-(2-ethylhexyl)- or Uvasorb K2A | 288254-16-0 |
| 64 | Merocyanine derivatives | |
| 65 | sterols (cholesterol, lanosterol, phytosterols), as described in WO0341675 | |
| 66 | mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes,) | |
| 67 | alpha-lipoic-acid as described in DE 10229995 | |
| 68 | synthetic organic polymers as described in EP 1371358, [0033]-[0041] | |
| 69 | phyllosilicates as described in EP 1371357 [0034]-[0037] | |
| 70 | silica compounds as described in EP1371356, [0033]-[0041] | |
| 71 | inorganic particles as described in DE10138496 [0043]-[0055] | |
| 72 | latex particles as described in DE10138496 [0027]-[0040] | |
| 73 | 1H-Benzimidazole-4,6-disulfonic acid, 2,2'-(1,4-phenylene)bis-, disodium salt; Bisimidazylate; Neo Heliopan APC | 180898-37-7 |
| 74 | Di-2-ethylhexyl-3,5-dimethoxy-4-hydroxy-benzalmalonate (Oxynex ST, EMD Chemicals, as described in US 20040247536) | |

The cosmetic or pharmaceutical preparations may be, for example, creams, gels, lotions, alcoholic and aqueous/alcoholic solutions, emulsions, wax/fat compositions, stick preparations, powders or ointments. In addition to the above-mentioned UV filters, the cosmetic or pharmaceutical preparations may contain further adjuvants as described below.

As water- and oil-containing emulsions (e.g. W/O, O/W, O/W/O and W/O/W emulsions or microemulsions) the preparations contain, for example, from 0.1 to 60 % by weight, preferably from 0.1 to 20 % by weight and especially from 0.5 to 10 % by weight, based on the total weight of the composition, of the aqueous dispersion according to the present invention, from 1 to 60 % by weight, especially from 5 to 50 % by weight and preferably from 10 to 35 % by weight, based on the total weight of the composition, of at least one oil component, from 0 to 30 % by weight, especially from 1 to 30 % by weight und preferably from 4 to 20 % by weight, based on the total weight of the composition, of at least one emulsifier, from 10 to 90 % by weight, especially from 30 to 90 % by weight, based on the total weight of the composition, of water, and from 0 to 88.9 % by weight, especially from 1 to 50 % by weight, of further cosmetically acceptable adjuvants.

The cosmetic or pharmaceutical compositions/preparations according to the present invention may also contain one or one more additional compounds like fatty alcohols, esters of fatty acids, natural or synthetic triglycerides including glyceryl esters and derivatives, pearlescent waxes: hydrocarbon oils: silicones or siloxanes, organosubstituted super-fatting agents, surfactants consistency regulators/thickeners and rheology modifiers, polymers, biogenic active ingredients, deodorising active ingredients, anti-dandruff agents, antioxidants, hydrotropic agents, preservatives and bacteria-inhibiting agents, perfume oils, colorants, polymeric beads or hollow spheres as spa enhancers.

Cosmetic or pharmaceutical formulations are contained in a wide variety of cosmetic preparations. There come into consideration, for example, especially the following preparations:
- skin-care preparations, e.g. skin-washing and cleansing preparations in the form of tablet-form or liquid soaps, soapless detergents or washing pastes,
- bath preparations, e.g. liquid (foam baths, milks, shower preparations) or solid bath preparations, e.g. bath cubes and bath salts;
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eyeshadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;
- fragrance preparations, e.g. fragrances (eau de Cologne, eau de toilette, eau de parfum, parfum de toilette, perfume), perfume oils or perfume creams;
- cosmetic hair-treatment preparations, e.g. hair-washing preparations in the form of shampoos and conditioners, hair-care preparations, e.g. pre-treatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colorants, preparations containing self-oxidising dyes, or natural hair colorants, such as henna or camomile.

### Presentation forms

The final formulations listed may exist in a wide variety of presentation forms, for example:
- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellant gas or pump-action spray) or an aerosol,
- in the form of a foam, or
- in the form of a paste.

Of special importance as cosmetic preparations for the skin are light-protective preparations, such as sun milks, lotions, creams, oils, sun blocks or tropicals, pretanning preparations or after-sun preparations, also skin-tanning preparations, for example self-tanning creams. Of particular interest are sun protection creams, sun protection lotions, sun protection milk and sun protection preparations in the form of a spray.

Of special importance as cosmetic preparations for the hair are the above-mentioned preparations for hair treatment, especially hair-washing preparations in the form of shampoos, hair conditioners, hair-care preparations, e.g. pre-treatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

A shampoo has, for example, the following composition: from 0.01 to 5 % by weight of a UV absorber composition according to the invention, 12.0 % by weight of sodium laureth-2-sulfate, 4.0 % by weight of cocamidopropyl betaine, 3.0 % by weight of sodium chloride, and water ad 100%.

Other typical ingredients in such formulations are preservatives, bactericides and bacteriostatic agents, perfumes, dyes, pigments, thickening agents, moisturizing agents, humectants, fats, oils, waxes or other typical ingredients of cosmetic and personal care formulations such as alcohols, poly-alcohols, polymers, electrolytes, organic solvents, silicon derivatives, emollients, emulsifiers or emulsifying surfactants, surfactants, dispersing agents, antioxidants, anti-irritants and anti-inflammatory agents etc.

The cosmetic and/or dermatological/pharmaceutical preparations according to the invention are distinguished by excellent protection of human skin against the damaging effect of sunlight.

Furthermore, the sunscreen compositions according to the present invention show beneficial effects relating to cell viability, the suppression of reactive oxygen species (ROS) as well as for the suppression of induction of gene expression related to skin aging and inflammation reactions.

### A. Preparation Examples

### A1. Preparation of a UV filter dispersion (rinse-off dispersants)

### Dispersion 1

### Slurry preparation:

In a beaker equipped with a stirrer 35.7g of Sodium Myreth Sulfate (aqueous paste with 70 wt-% active matter) are dissolved in 801.8g of deionized water. 275g of decyl glucoside (50 wt-% active in water) are added, and the solution is adjusted to pH = 4.5 with 62.5g of a 20 wt-% aqueous solution of citric acid. Then 25g of Simethicone (mixture of Dimethicone with an average chain length of 200 to 350 dimethylsiloxane units and hydrated silica, 20 wt-% in water) are added. After homogenization 1250g of the compound of formula 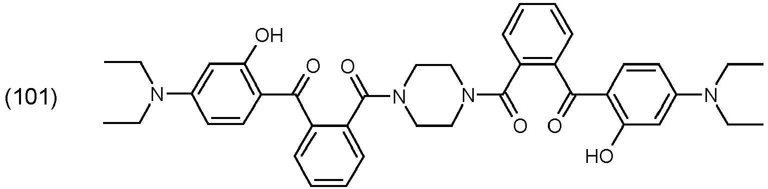 are added.

### Pre-Grinding process:

The slurry is passed twice through a colloid mill (Fryma) for homogenization. Then the colloid mill is switched to circulation mode and the slurry is wet-grinded for five minutes.

### Fine-Grinding process:

The mill is filled with 440 ml Draison grinding beads having a diameter of 0.3 to 0.4 mm. The pre-grinded slurry is transferred into a beaker equipped with a IKA stirrer. A Watson-Marlow pump is used for slurry circulation. The grinding compartment is filled with the slurry without running the mill, at low speed of the Watson-Marlow pump. The mill is switched on as soon as the initial pressure in the mill increases.

The fine grinding is carried out with the following parameters:

| | |
|---|---|
| Rotation speed of the mill: | 2389 rpm |
| Rotation speed of the pump: | 20-40 rpm |
| IKA stirrer speed: | 80 rpm |
| Tempₘₐₓ: | 48°C |
| Pressure: | 0.2 - 0.5 bar |
| Current: | 4,5-6A |
| Power uptake for grinding: | initial: 1420 W; at the end of the process: 550-500 W |
| Energy consumption: | 3000 W/h |

After 5h the fine grinding is finished. The mill is discharged, and the final product is stored at room temperature.

UV filter dispersion 1 contains rinse-off surfactants as grinding aids.

Using the same procedure, the UV filter dispersions 2-12 are prepared. Optionally, a preservation system is added to the UV filter dispersion.

The particle size of the micronized UV filter dispersions is characterized with a Mastersizer 2000 (Malvern Instruments) equipped with a wet dispersion unit. This instrument measures the particle size distribution of the dispersed particles by laser diffraction.

The d50 value is a measure for the average particle size of the micronized UV filter particles.

The compositions are summarised as follows, wherein the dispersions No. 5-7 and 10-12 are dispersions according to the invention and the dispersions No. 1-4 and 8-9 are comparative dispersions:

| UV dispersion No. (inventive: dispersions No. 5-7 and 10-12: comparative: dispersions No. 1-4 and 8-9) | | 1 | 2 | 3 | 4 | 5 | 6 | 7 | 8 | 9 | 10 | 11 | 12 |
|---|---|---|---|---|---|---|---|---|---|---|---|---|---|
| UV Filter [wt-%] | (a₁) compound of formula (1c) | 50 | | 50 | | 50 | 50 | 50 | | 50 | 50 | 50 | 50 |
| | (a₂) compound of formula (2b) | | 50 | | | | | | | | | | |
| | (a₃) compound of formula (3c) | | | | 40 | | | | | | | | |
| | (a₄) compound of formula (4b) | | | | | | | | 50 | | | | |
| Dispersant [active matter wt-%] | Sodium myreth sulfate | 5 | | | | | | | | | | | |
| | Alkyl polyglucoside | 1 | 6,5 | | | | | | | | | | |
| | Polyglyceryl 10 Laurate | | | 12 | 8 | | | | 10 | | | | |
| | Sodium C12-18 Alkyl Sulfosuccinate | | | | | | | | | 4 | | | |
| | Sodium Cocoyl Glutamate | | | | | | 5 | 5 | | | | | |
| | Disodium Cocoyl Glutamate | | | | | | | | | | 5 | 5 | 5 |
| | Sodium Stearoyl Glutamate | | | | | 5 | | | | | | | |
| | Sodium Surfactin | | | | | | 0,5 | 0,5 | | 1 | 0,5 | 0,5 | 0,5 |
| | Hydrolyzed Wheat Protein | | | | | 0,3 | | | | | | | |
| Defoamer [active matter, wt-%] | Simethicone | 0,2 | 0,2 | | | 0,2 | | | | | | | |
| | Citric acid | 0,4 | | | 0,1 | | | | | | | | |
| Additive for pH adjustment [wt-%] | Citric acid | 0,4 | | | 0,1 | | | | | | | | |
| | Caustic soda | | 0,1 | | | | | | | | | | |
| Preservative [active matter, wt-%]: | Phenoxyethanol | | | 1 | 1 | | 1 | | | | | | |
| | Caprylyl glycole | | | | | | | 1 | | | 1 | 1 | 1 |
| | Benzyl alcohol (and) dehydroacetic acid | | | | | | | | | 1 | | | |
| Thickener [wt-%] | Xanthan Gum | | | | | | | | | | | 0,2 | 0,4 |
| Water | | ad 100 | ad 100 | ad 100 | ad 100 | ad 100 | ad 100 | ad 100 | ad 100 | ad 100 | ad 100 | ad 100 | ad 100 |
| PSD: d50 [nm] | | 12q | 135 | 130 | 122 | 126 | 119 | 121 | 124 | 115 | 121 | 121 | 121 |

The UV filter dispersions 1-12 contain only mild leave-on dispersants and are suitable for the formulation of mild cosmetic sunscreens and skin care consumer products.

### Thickening of the UV filter dispersions:

Thickening of dispersions 1 - 5, 8 and 9 with polyvinylpyrrolidone:
5g of polyvinylpyrrolidone are dissolved in 5g water (preferably 24h bevor the grinding) and mixed into the slurry with a stirrer.

### Viscosity of the UV filter dispersions:

The viscosity of the UV filter dispersions is analysed in a Contrave Rheomat RM115 viscosimeter, which determines the flow behaviour and viscosity of the sample.

| Sample Thickener | Dispersion 1 w/o thickener | Dispersion 1 PVP | Dispersion 6 w/o thickener | Dispersion 7 w/o thickener |
|---|---|---|---|---|
| Viscosity [mPa s] at 80rpm at 25°C, increasing shear rate | 248 | 404 | 560 | 513 |
| Viscosity [mPa s] at 500rpmat 25°C, constant shear rate | 72 | 119 | 165 | 157 |

The data demonstrate that the flow behaviour of the dispersions 6 and 7 based on mild dispersants as grinding aids is similar to dispersion 2 with added PVP, and do not require a thickener.

### B. Formulation examples

The below formulation efficacy (SPF, UVA-PF) was assessed with BASF Simulator (www.basf.com/sunscreensimulator; Herzog, B.; Osterwalder, U. In Silico Determination of Topical Sun Protection. Cosm Sci Tech. 2011; 62: 1-8).

| Example F1: Cream Gel Carbopol | | | |
|---|---|---|---|
| Phase | Ingredients | INCl | % by weight |
| A | Cetiol^{®} AB | C12-15 Alkyl Benzoate | 12.00 |
| | DUB DIS | Diisopropyl Sebacate | 10.00 |
| | Uvinul^{®} A Plus | Diethylamino Hydroxybenzoyl Hexyl Benzoate | 5.00 |
| | Tinosorb^{®} S | Bis-Ethylhexyloxyphenol Methoxyphenyl Triazine | 2.00 |
| | Uvinul^{®} T 150 | Ethylhexyl Triazone | 1.50 |
| B | Water, demin. | Aqua | 27.30 |
| | Butylene Glycol | Butylene Glycol | 4.00 |
| | Glycerin | Glycerin | 3.00 |
| | Tinocare GL | Sclerotium Gum | 3.00 |
| | Edeta^{®} B Powder | Tetrasodium EDTA | 0.20 |
| | Rheocare TTA | Acrylates Copolymer | 0.30 |
| | Carbopol Ultrez 10 Polymer | Carbomer | 0.20 |
| | Pemulen TR-2 Polymeric Emulsifier | Acrylates/C10-30 Alkyl Acrylate Crosspolymer | 0.15 |
| | Keltrol RD | Xanthan Gum | 0.15 |
| C | Water, demin. | Aqua | 5.00 |
| | Eusolex 232 | Phenylbenzimidazole Sulfonic Acid | 3.00 |
| | AMP-Ultra PC 2000 | Aminomethyl Propanol | 0.20 |
| D | Ethanol | Alcohol | 5.00 |
| E | Siclone SR-5 | C13-C16 Isoparaffin, C12-C14 Isoparaffin, C13-C15 Alkane | 4.00 |
| | Micropearl M305 | Methylmethacrylate Crosspolymer | 3.00 |
| | UV dispersion 1 or 2 or 3 or 4 or 5 or 6 or 7 or 8 or 9 or 10 or 11 or 12 according to Example A1 | | 10.00 |
| | Geogard 221 | Benzyl alcohol (and) dehydroacetic acid | 1.00 |
| | Perfume | Alpha-Isomethylionon, Benzylalkohol, Benzylcinnamat, Benzylsalicylat, Citronellol, Coumarin, Eugenol, Geraniol, Hexylcinnamal, Linalool, Limonen | qs |
| | | SPF | 35.8 |
| | | UVA-PF | 26.1 |

| Example F2: Cream Gel Carbopol | | | |
|---|---|---|---|
| Phase | Ingredients | INCI | % by weight |
| A | Cetiol^{®} AB | C12-15 Alkyl Benzoate | 12.00 |
| | DUB DIS | Diisopropyl Sebacate | 10.00 |
| | Uvinul^{®} A Plus | Diethylamino Hydroxybenzoyl Hexyl Benzoate | 6.00 |
| | Tinosorb^{®} S | Bis-Ethylhexyloxyphenol Methoxyphenyl Triazine | 2.00 |
| | Uvinul^{®} T 150 | Ethylhexyl Triazone | 1.50 |
| B | Water, demin. | Aqua | 26.30 |
| | Butylene Glycol | Butylene Glycol | 4.00 |
| | Glycerin | Glycerin | 3.00 |
| | Tinocare GL | Sclerotium Gum | 3.00 |
| | Edeta^{®} B Powder | Tetrasodium EDTA | 0.20 |
| | Rheocare TTA | Acrylates Copolymer | 0.30 |
| | Carbopol Ultrez 10 Polymer | Carbomer | 0.20 |
| | Pemulen TR-2 Polymeric Emulsifier | Acrylates/C10-30 Alkyl Acrylate Crosspolymer | 0.15 |
| | Keltrol RD | Xanthan Gum | 0.15 |
| C | Water, demin. | Aqua | 5.00 |
| | Eusolex 232 | Phenylbenzimidazole Sulfonic Acid | 3.00 |
| | AMP-Ultra PC 2000 | Aminomethyl Propanol | 0.20 |
| D | Ethanol | Alcohol | 5.00 |
| E | Siclone SR-5 | C13-C16 Isoparaffin, C12-C14 Isoparaffin, C13-C15 Alkane | 4.00 |
| | Micropearl M305 | Methylmethacrylate Crosspolymer | 3.00 |
| | UV dispersion 1 or 2 or 3 or 4 or 5 or 6 or 7 or 8 or 9 or 10 or 11 or 12 according to Example A1 | | 10.00 |
| | Tinosorb^{®}M | Methylene Bis-Benzotriazolyl Tetramethylbutylphenol (nano), Aqua, Decyl Glucoside, Propylene Glycol, Xanthan Gum | 10.00 |
| | Geogard 221 | Benzyl alcohol (and) dehydroacetic acid | 1.00 |
| | Perfume | Alpha-Isomethylionon, Benzylalkohol, Benzylcinnamat, Benzylsalicylat, Citronellol, Coumarin, Eugenol, Geraniol, Hexylcinnamal, Linalool, Limonen | qs |
| SPF | | | 50.3 |
| UVA-PF | | | 43.8 |

| Example F3: Prisma SP | | | F3-A | F3-B |
|---|---|---|---|---|
| Phase | Ingredients | INCI | % by weight | % by weight |
| A | Eumulgin^{®} Prisma | Disodium Cetearyl Sulfosuccinate | 1.500 | 1.500 |
| | Cutina^{®} HVG | Hydrogenated Vegetable Glycerides | 2.000 | 2.000 |
| | Lanette^{®} O | Cetearyl Alcohol | 2.000 | 2.000 |
| | Cutina^{®} PES | Pentaerythrityl Distearate | 1.000 | 1.000 |
| | Cetiol^{®} B | Dibutyl Adipate | 10.000 | 10.000 |
| | Cetiol^{®} AB | C12-15 Alkyl Benzoate | 5.000 | 5.000 |
| | Cetiol^{®} CC | Dicaprylyl Carbonate | 3.000 | 3.000 |
| | Uvinul^{®} A Plus | Diethylamino Hydroxybenzoyl Hexyl Benzoate | 4.500 | 4.500 |
| | Uvinul^{®} T 150 | Ethylhexyl Triazone | 2.000 | 2.000 |
| B | Water, demin. | Aqua | 36.400 | 30.400 |
| | Glycerin | Glycerin | 3.000 | 3.000 |
| | Cosmedia^{®} SP | Sodium Polyacrylate | 0.700 | 0.700 |
| | Rheocare^{®} XG (Europe only) | Xanthan Gum | 0.200 | 0.200 |
| | Edeta^{®} BD | Disodium EDTA | 0.200 | 0.200 |
| C | Water, demin. | Aqua | 6.000 | 6.000 |
| | Eusolex 232 | Phenylbenzimidazole Sulfonic Acid | 1.000 | 1.000 |
| | Tris Amino Ultra Pur | Tromethamine | 0.500 | 0.500 |
| D | Xiameter PMX-0246 Cyclohexasiloxane | Cyclohexasiloxane, Cyclopentasiloxane | 2.000 | 2.000 |
| | Techpolymer MBP 8 | Polymethyl Methacrylate | 1.000 | 1.000 |
| E | Tinosorb^{®} S Aqua | Aqua, Bis-Ethylhexyloxyphenol Methoxyphenyl Triazine, Polymethyl Methacrylate, Sodium Laureth Sulfate, Aminomethyl Propanol | 10.000 | 10.000 |
| | UV dispersion 1 or 2 or 3 or 4 or 5 or 6 or 7 or 8 or 9 or 10 or 11 or 12 according to Example A1 | | 8.000 | 8.000 |
| | Triasorb^{®} | Phenylene bis-diphenyltriazine, PPG-1 PEG-9lauryl glycol ether, benzoic acid | | 6.000 |
| | Preservative | | qs | qs |
| SPF | | | 28.6 | 33.6 |
| UVA-PF | | | 24.6 | 29.7 |

| Example F4: Lotion O-W SG | | | |
|---|---|---|---|
| Phase | Ingredients | INCI | % by weight |
| A | Eumulgin^{®} SG | Sodium Stearoyl Glutamate | 2.00 |
| | Tegosoft XC | Phenoxyethyl Caprylate | 8.00 |
| | Cetiol^{®} B | Dibutyl Adipate | 5.00 |
| | Finsolv EB | Ethylhexyl Benzoate | 5.00 |
| | Lanette^{®} 18 | Stearyl Alcohol | 1.00 |
| | Uvinul^{®} A Plus | Diethylamino Hydroxybenzoyl Hexyl Benzoate | 7.00 |
| | Uvinul^{®} T 150 | Ethylhexyl Triazone | 3.00 |
| | Tinosorb^{®} S | Bis-Ethylhexyloxyphenol Methoxyphenyl Triazine | 4.00 |
| B | Water, demin. | Aqua | 36.00 |
| | Edeta^{®} BD | Disodium EDTA | 0.20 |
| | Rheocare^{®} XG | Xanthan Gum | 0.50 |
| C | UV dispersion 1 or 2 or 3 or 4 or 5 or 6 or 7 or 8 or 9 or 10 or 11 or 12 according to Example A1 | | 20.00 |
| | Tinosorb^{®} W PGL | Methylene Bis-Benzotriazolyl Tetramethylbutylphenol (nano), Aqua, Polyglyceryl-10 laurate, butylene Glycol, | 8.00 |
| D | Perfume | Parfum | 0.10 |
| | Preservative | | 0.20 |
| SPF | | | 54.4 |
| UVA-PF | | | 59 |

| Example F5: SWOP lotion | | | |
|---|---|---|---|
| Phase | Ingredients | INCI | % by weight |
| A | Dehymuls^{®} PGPH | Polyglyceryl-2 Dipolyhydroxystearate | 4.00 |
| | Cetiol^{®} B | Dibutyl Adipate | 15.00 |
| | Cetiol^{®} AB | C12-15 Alkyl Benzoate | 10.00 |
| | Lanette^{®} O | Cetearyl Alcohol | 2.50 |
| | Uvinul^{®} A Plus | Diethylamino Hydroxybenzoyl Hexyl Benzoate | 5.2 |
| | Uvinul^{®} T 150 | Ethylhexyl Triazone | 3.20 |
| | Tinosorb^{®} S | Bis-Ethylhexyloxyphenol Methoxyphenyl Triazine | 2.50 |
| B | Water, demin. | Aqua | 33.95 |
| | Glycerin | Glycerin | 3.00 |
| | Plantapon^{®} LGC SORB | Sodium Lauryl Glucose Carboxylate (and) Lauryl Glucoside | 1.50 |
| | Cosmedia^{®} SP | Sodium Polyacrylate | 0.80 |
| | Edeta^{®} BD | Disodium EDTA | 0.20 |
| | Keltrol CG-RD | Xanthan Gum | 0.15 |
| C | Tinosorb^{®} A2B | Tris-Biphenyl Triazine (nano), Aqua, Decyl Glucoside, Butylene Glycol, Disodium Phosphate, Xanthan Gum | 8.00 |
| | UV dispersion 1 or 2 or 3 or 4 or 5 or 6 or 7 or 8 or 9 or 10 or 11 or 12 according to Example A1 | | 8.00 |
| | Techpolymer MB 8C | Polymethyl Methacrylate | 2.00 |
| | Preservative | | qs |
| | Perfume | Parfum | qs |
| SPF | | | 60.2 |
| UVA-PF | | | 26.7 |

| Example F6: Suco Prisma | | | |
|---|---|---|---|
| Phase | Ingredients | INCI | % by weight |
| A | Emulgade^{®} Sucro Plus | Sucrose Polystearate, Cetyl Palmitate | 3.00 |
| | Eumulgin^{®} Prisma | Disodium Cetearyl Sulfosuccinate | 1.00 |
| | Lanette^{®} O | Cetearyl Alcohol | 1.00 |
| | DUB DIS | Diisopropyl Sebacate | 10.00 |
| | Exceparl LM-LC | Lauryl Lactate | 5.00 |
| | Uvinul^{®} A Plus | Diethylamino Hydroxybenzoyl Hexyl Benzoate | 5.00 |
| | Uvinul^{®} T 150 | Ethylhexyl Triazone | 3.00 |
| B | Water, demin. | Aqua | 52.10 |
| | Glycerin | Glycerin | 2.00 |
| | Cosmedia^{®} SP | Sodium Polyacrylate | 1.00 |
| | Keltrol CG-RD | Xanthan Gum | 0.20 |
| | Edeta^{®} BD | Disodium EDTA | 0.20 |
| C | Tinosorb^{®} M | Methylene Bis-Benzotriazolyl Tetramethylbutylphenol (nano), Aqua, Decyl Glucoside, Propylene Glycol, Xanthan Gum | 7.00 |
| | UV dispersion 1 or 2 or 3 or 4 or 5 or 6 or 7 or 8 or 9 or 10 or 11 or 12 according to Example A1 | | 6.00 |
| D | Cetiol^{®} Ultimate | Undecane, Tridecane | 1.50 |
| | Techpolymer MBP 8 | Polymethyl Methacrylate | 1.00 |
| | Orgasol Caresse | Polyamide-5 | 1.00 |
| | Preservative | | qs |
| SPF | | | 30.3 |
| UVA-PF | | | 24.4 |

| Example F7: PL68-50-Prisma | | | |
|---|---|---|---|
| Phase | Ingredients | INCI | % by weight |
| A | Emulgade^{®} PL 68/50 | Cetearyl Glucoside, Cetearyl Alcohol | 4.00 |
| | Eumulgin^{®} Prisma | Disodium Cetearyl Sulfosuccinate | 1.00 |
| | Lanette^{®} O | Cetearyl Alcohol | 1.00 |
| | Cutina^{®} PES | Pentaerythrityl Distearate | 1.00 |
| | Cetiol^{®} B | Dibutyl Adipate | 8.00 |
| | Cetiol^{®} CC | Dicaprylyl Carbonate | 3.00 |
| | Uvinul^{®} N 539T | Octocrylene | 10.00 |
| | Parsol 1789 | Butyl Methoxydibenzoylmethane | 5.00 |
| | Neo Heliopan OS | Ethylhexyl Salicylate | 5.00 |
| | Tinosorb^{®} S | Bis-Ethylhexyloxyphenol Methoxyphenyl Triazine | 1.00 |
| B | Water, demin. | Aqua | 38.70 |
| | Glycerin | Glycerin | 3.00 |
| | Mais PO4 PH "B" | Distarch Phosphate | 1.00 |
| | Keltrol CG-RD | Xanthan Gum | 0.30 |
| C | Tinosorb^{®} A2B | Tris-Biphenyl Triazine (nano), Aqua, Decyl Glucoside, Butylene Glycol, Disodium Phosphate, Xanthan Gum | 6.00 |
| | UV dispersion 1 or 2 or 3 or 4 or 5 or 6 or 7 or 8 or 9 or 10 or 11 or 12 according to Example A1 | | 6.00 |
| | Tinosorb^{®} M | Methylene Bis-Benzotriazolyl Tetramethylbutylphenol (nano), Aqua, Decyl Glucoside, Propylene Glycol, Xanthan Gum | 2.00 |
| D | Xiameter PMX-0245 Cyclopentasiloxane | Cyclopentasiloxane | 2.00 |
| | Orgasol 2002 EXD NAT COS | Nylon-12 | 2.00 |
| | Perfume | Parfum | qs |
| | Preservative | | qs |
| SPF | | | 54.6 |
| UVA-PF | | | 27.9 |

| Example F8: GTC Up | | | |
|---|---|---|---|
| Phase | Ingredients | INCI | % by weight |
| A | Cetiol^{®} CC | Dicaprylyl Carbonate | 5.00 |
| | Cetiol^{®} C 5C | Coco-Caprylate/Caprate | 3.00 |
| | Uvinul^{®} MC 80 | Ethylhexyl Methoxycinnamate | 10.00 |
| | Uvinul^{®} A Plus | Diethylamino Hydroxybenzoyl Hexyl Benzoate | 8.00 |
| | Neo Heliopan OS | Ethylhexyl Salicylate | 5.00 |
| | Uvinul^{®} T 150 | Ethylhexyl Triazone | 2.50 |
| B | Water, demin. | Aqua | 31.00 |
| | Tinocare^{®} GL | Sclerotium Gum | 2.50 |
| | Tinovis^{®} GTC UP | Acrylates/Beheneth-25 Methacrylate Copolymer | 2.00 |
| C | Sodium Hydroxide (30% solution) | Sodium Hydroxide | qs |
| D | Tinosorb^{®} M | Methylene Bis-Benzotriazolyl Tetramethylbutylphenol (nano), Aqua, Decyl Glucoside, Propylene Glycol, Xanthan Gum | 4.00 |
| | UV dispersion 1 or 2 or 3 or 4 or 5 or 6 or 7 or 8 or 9 or 10 or 11 or 12 according to Example A1 | | 10.00 |
| E | Ethanol | Alcohol | 10.00 |
| | Xiameter PMX-0246 Cyclohexasiloxane | Cyclohexasiloxane, Cyclopentasiloxane | 4.00 |
| | Orgasol 4000 EXD NAT COS Caresse | Nylon-6/12 | 2.00 |
| | Tospearl A145 | Polymethylsilsesquioxane | 1.00 |
| SPF | | | 57.40 |
| UVA-PF | | | 21.00 |

| Example F9: Ace prisma | | | |
|---|---|---|---|
| Phase | Ingredients | INCI | % by weight |
| A | Eumulgin^{®} Prisma | Disodium Cetearyl Sulfosuccinate | 0.50 |
| | Cetiol^{®} CC | Dicaprylyl Carbonate | 11.00 |
| | Cetiol^{®} B | Dibutyl Adipate | 8.00 |
| | Cetiol^{®} Ultimate | Undecane, Tridecane | 5.00 |
| | Cetiol^{®} C 5 | Coco-Caprylate | 8.00 |
| | Preservative | | qs |
| | Tinosorb^{®} S | Bis-Ethylhexyloxyphenol Methoxyphenyl Triazine | 2.50 |
| | Uvinul^{®} T 150 | Ethylhexyl Triazone | 2.00 |
| B | Water, demin. | Aqua | 23.50 |
| | Glycerin | Glycerin | 3.00 |
| | Keltrol CG-RD | Xanthan Gum | 0.30 |
| | Edeta^{®} BD | Disodium EDTA | 0.20 |
| C | Cosmedia^{®} Ace | Sodium Polyacrylate, Dicaprylyl Carbonate, Polyglyceryl-3 Caprate | 1.00 |
| D | Sodium Hydroxide (30% solution) | Sodium Hydroxide | qs |
| E | Tinosorb^{®} M | Methylene Bis-Benzotriazolyl Tetramethylbutylphenol (nano), Aqua, Decyl Glucoside, Propylene Glycol, Xanthan Gum | 12.00 |
| | Tinosorb^{®} A2B | Tris-Biphenyl Triazine (nano), Aqua, Decyl Glucoside, Butylene Glycol, Disodium Phosphate, Xanthan Gum | 8.00 |
| | UV dispersion 1 or 2 or 3 or 4 or 5 or 6 or 7 or 8 or 9 or 10 or 11 or 12 according to Example A1 | | 10.00 |
| F | Lipofructyl^{™} Argan LS 9779 | Argania Spinosa Kernel Oil | 2.50 |
| | Melhydran^{™} LS 4420 | Honey Extract | 2.50 |
| | Perfume | Parfum | qs |
| SPF | | | 60.90 |
| UVA-PF | | | 26.40 |

| Example F10: Ace | | | |
|---|---|---|---|
| Phase | Ingredients | INCI | % by weight |
| A | Cetiol^{®} C 5 | Coco-Caprylate | 3.00 |
| | Uvinul^{®} MC 80 | Ethylhexyl Methoxycinnamate | 10.00 |
| | Uvinul^{®} A Plus | Diethylamino Hydroxybenzoyl Hexyl Benzoate | 5.50 |
| | Neo Heliopan OS | Ethylhexyl Salicylate | 3.00 |
| | Preservative | | qs |
| B | Water, demin. | Aqua | 31.50 |
| | 1,2 Butanediol | Butylene Glycol | 2.50 |
| C | Cosmedia^{®} Ace | Sodium Polyacrylate, Dicaprylyl Carbonate, Polyglyceryl-3 Caprate | 1.00 |
| D | Tinosorb^{®} M | Methylene Bis-Benzotriazolyl Tetramethylbutylphenol (nano), Aqua, Decyl Glucoside, Propylene Glycol, Xanthan Gum | 4.00 |
| | Triasorb^{®} | Phenylene bis-diphenyltriazine, PPG-1 PEG-9lauryl glycol ether, benzoic acid | 4.00 |
| | UV dispersion 1 or 2 or 3 or 4 or 5 or 6 or 7 or 8 or 9 or 10 or 11 or 12 according to Example A1 | | 20.00 |
| E | Ethanol | Alcohol | 10.00 |
| | Cetiol^{®} Ultimate | Undecane, Tridecane | 4.00 |
| | Techpolymer ACP-8C | Acrylates/Ethylhexyl Acrylate Crosspolymer | 1.50 |
| | Sodium Hydroxide (30% solution) | Sodium Hydroxide | qs |
| SPF | | | 51.90 |
| UVA-PF | | | 31.10 |

| Example F11: ADE | | | |
|---|---|---|---|
| Phase | Ingredients | INCI | % by weight |
| A | Cosmedia^{®} DC | Hydrogenated Dimer Dilinoleyl/Dimethylcarbonate Copolymer | 2.00 |
| | Cetiol^{®} C 5 | Coco-Caprylate | 5.00 |
| | Uvinul^{®} A Plus B | Ethylhexyl Methoxycinnamate, Diethylamino Hydroxybenzoyl Hexyl Benzoate | 6.0 |
| | Cetiol^{®} B | Dibutyl Adipate | 10.00 |
| B | Water, demin. | Aqua | 58.8 |
| | Edeta^{®} BD | Disodium EDTA | 0.20 |
| C | Tinovis^{®} ADE | Sodium Acrylates Copolymer, Hydrogenated Polydecene, PPG-1 Trideceth-6 | 1.00 |
| D | Tinosorb^{®} M | Methylene Bis-Benzotriazolyl Tetramethylbutylphenol (nano), Aqua, Decyl Glucoside, Propylene Glycol, Xanthan Gum | 8.0 |
| | UV dispersion 1 or 2 or 3 or 4 or 5 or 6 or 7 or 8 or 9 or 10 or 11 or 12 according to Example A1 | | 8.0 |
| E | Perfume | Parfum | qs |
| | Protectol^{®} PE | Phenoxyethanol | 1.00 |
| SPF | | | 26.4 |
| UVA-PF | | | 14.4 |

| Example F12: | | | |
|---|---|---|---|
| Phase | Trade name | INCI | % by weight |
| A | Emulgade PL 68/50 | Ceateryl Glucoside (nad) Cetearyl Alcohol | 4.00 |
| | Eumulgin^{®} prisma | Disodium Cetearyl Sulfosuccinate | 1.00 |
| | Cetiol AB | C12-15 Alkyl Benzoate | 8.00 |
| | Cetiol B | Dibutyl Adipate | 8.00 |
| | Cetiol C 5 | Coco-Caprylate | 4.00 |
| | Cutina PES | Pentaerythrityl Distearate | 1.00 |
| | Tinosorb^{®} S | Bis-Ethylhexyloxyphenol Methoxyphenyl Triazine | 2.00 |
| B | Water | Aqua | 46.25 |
| | Glycerin 85% | Glycerin | 3.00 |
| | EDTA BD | Di Na EDTA | 0.20 |
| | Rheocare XG | Xanthan Gum | 0.40 |
| | Protectol PE | Phenoxyethanol | 1.00 |
| | Rheocare C Plus | Carbomer | 0.15 |
| C | UV dispersion 1 or 2 or 3 or 4 or 5 or 6 or 7 or 8 or 9 or 10 or 11 or 12 according to Example A1 | | 4.00 |
| | Tinosorb^{®} S Aqua | Aqua, Bis-Ethylhexyloxyphenol Methoxyphenyl Triazine, Polymethyl Methacrylate, Sodium Laureth Sulfate, Aminomethyl Propanol | 5.00 |
| | Spheron 1500 | Silica | 1.00 |
| | Orgasol carresseCaresse | Polyamide-5 | 2.00 |
| | 50% LösSolsol. Neutrol TE | Tetrahydroxypropyl Ethylenediamine | 1.00 |
| | 11.7 | | |
| | | | |
| Manufacturing instruction: | | | |
| Heat up Part A to 85°C | | | |
| Let swell Rheocare C Plus into water. | | | |
| When homogenous add the rest of part B and heat to 80°C. | | | |
| Heat up Part B to 80°C | | | |
| Add Part A into B under Turrax | | | |
| Cool down to RT | | | |
| Add ingredients of Part C under stirring | | | |
| Continue stirring for a while | | | |

| Example F13: | | | |
|---|---|---|---|
| Phase | Trade name | INCI | % by weight |
| A | Emulgade PL 68/50 | Ceateryl Glucoside (nad) Cetearyl Alcohol | 4.00 |
| | Eumulgin^{®} prisma | Disodium Cetearyl Sulfosuccinate | 1.50 |
| | Cetiol AB | C12-15 Alkyl Benzoate | 8.00 |
| | Cetiol B | Dibutyl Adipate | 8.00 |
| | DUB DIS | Diisopropyl Sebacate | 5.00 |
| | Cetiol C 5 | Coco-Caprylate | 4.00 |
| | Cutina PES | Pentaerythrityl Distearate | 1.00 |
| | Uvinul^{®} A Plus | DBT | 3.00 |
| | Uvinul^{®} T 150 | Ethylhexyl Triazone | 2.00 |
| | Tinosorb^{®} S | Bis-Ethylhexyloxyphenol Methoxyphenyl Triazine | 2.00 |
| B | Water | Aqua | 25.45 |
| | Glycerin 85% | Glycerin | 3.00 |
| | EDTA BD | Di Na EDTA | 0.20 |
| | Rheocare XG | Xanthan Gum | 0.40 |
| | Protectol PE | Phenoxyethanol | 1.00 |
| | Rheocare C Plus | Carbomer | 0.15 |
| C | Tinosorb^{®} S Aqua | Aqua, Bis-Ethylhexyloxyphenol Methoxyphenyl Triazine, Polymethyl Methacrylate, Sodium Laureth Sulfate, Aminomethyl Propanol | 7.50 |
| | UV dispersion 1 or 2 or 3 or 4 or 5 or 6 or 7 or 8 or 9 or 10 or 11 or 12 according to Example A1 | UV dispersion 1 or 2 or 3 or 4 or 5 or 6 or 7 or 8 or 9 or 10 or 11 or 12 according to Example A1 | 20.00 |
| | Spheron 1500 | Silica | 1.00 |
| | Orgasol Caresse | Polyamide-5 | 2.00 |
| | 50% SolSol.Neutrol TE | Tetrahydroxypropyl Ethylenediamine | 0.80 |
| SPF | | | 48.3 |
| Manufacturing instruction: | | | |
| Heat up Part A to 85°C | | | |
| Let swell Rheocare C Plus into water. | | | |
| When homogenous add the rest of part B and heat to 80°C. | | | |
| Heat up Part B to 80°C | | | |
| Add Part A into B under Turrax | | | |
| Cool down to RT | | | |
| Add ingredients of Part C under stirring | | | |
| Continue stirring for a while | | | |

| Example F14: | | |
|---|---|---|
| Trade name | INCI | % by weight |
| Eumulgin^{®} B 2 | Ceteareth-20 | 1.50 |
| Eumulgin^{®} Prisma | Disodium Cetearyl Sulfosuccinate | 2.50 |
| Cetiol B | Dibutyl Adipate | 11.00 |
| DUB DIS | Diisopropyl Sebacate | 7.00 |
| Uvinul^{®} A Plus | Diethylamino Hydroxybenzoyl Hexyl Benzoate | 8.00 |
| Uvinul^{®} T 150 | Ethylhexyl Triazone | 3.00 |
| Tinosorb^{®} S | Bis-Ethylhexyloxyphenol Methoxyphenyl Triazine | 2.00 |
| Water | Aqua | 21.40 |
| EDTA BD | Di Na EDTA | 0.20 |
| Rheocare XG | Xanthan Gum | 0.50 |
| Protectol PE | Phenoxyethanol | 1.00 |
| Eusolex 232 | Phenylbenzimidazole Sulfonic Acid | 1.00 |
| Water | Aqua | |
| 50% Sol. Neutrol TE | Tetrahydroxypropyl Ethylenediamine | 2.90 |
| UV dispersion 1 or 2 or 3 or 4 or 5 or 6 or 7 or 8 or 9 or 10 or 11 or 12 according to Example A1 | | 20 |
| Tinosorb^{®} S Aqua | Aqua, Bis-Ethylhexyloxyphenol Methoxyphenyl Triazine, Polymethyl Methacrylate, Sodium Laureth Sulfate, Aminomethyl Propanol | 10.00 |
| Techpolymer MBP-8 | Polymethyl Methacrylate | 2.00 |
| | SPF | 129 |

### C. Application Examples

### Investigation of the different protective effects of a conventional sunscreen formulation and a sunscreen formulation with comparable sun protection factor (SPF) but also containing the UV filter of formula (1c)

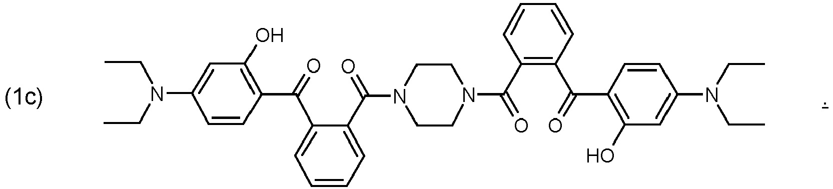

Keratinocyte cell cultures are employed which are irradiated through UV transparent PMMA substrates on which the different sunscreens or a placebo formulation are distributed, respectively.

Four types of endpoints are used:
1. Cell viability,
2. Reactive oxygen species (ROS) formation,
3. DNA damage, and
4. Gene induction.

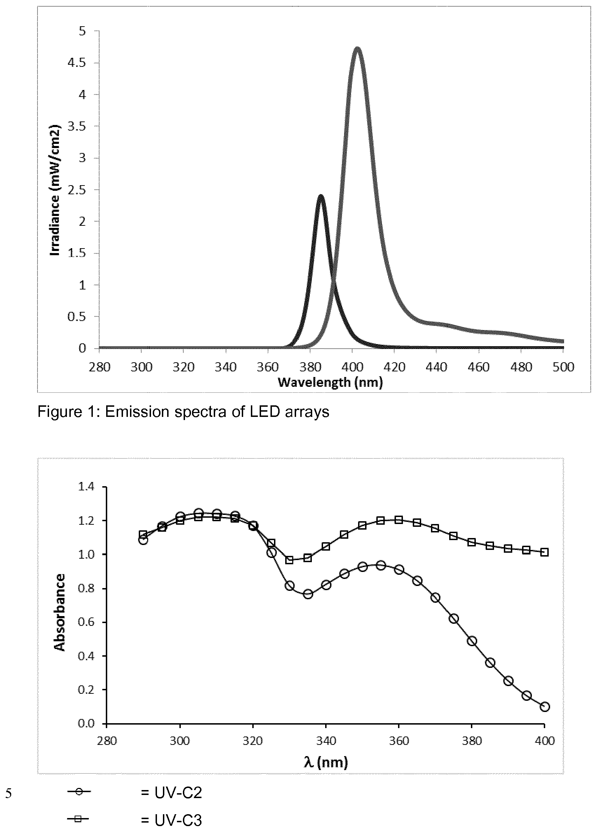

As light sources two light emitting diode (LED) arrays are used (Loctite), one emitting at 385 nm and the other at 405 nm. The emission spectra are shown in Figure 1. The 405 nm LED array shows considerable emission in the visible light rang, more than 76%, in contrast to the 385 nm LED array which has about 95% of its emission in the UVA range and only 5% in the visible.

Model sunscreens are prepared containing the filter compositions shown in Table 3. The SPF is calculated using the BASF Sunscreen Simulator software. The UV spectra of film absorbance simulated with the same program are shown in Figure 2.

| Table 3: Sunscreens used in the study | | | | |
|---|---|---|---|---|
| Formulation | UV-C1a « Placebo» | UV-C1b « Glycerol » | UV-C2 « Traditional » | UV-C3 « Trad + C1332 » |
| | - | | UVAPF/SPF = 0.34 | UVAPF/SPF = 0.83 |
| UV filter components | No UV filters | Glycerol | 1.5% Uvinul^{®} T150 | 1.0% Uvinul^{®} T150 |
| | | No UV filters | | |
| | | | 2.0% PBSA | 1.5% PBSA |
| | | | 2.0% Uvinul^{®} A Plus | 2.0% Uvinul^{®} A Plus |
| | | | | 10.0% of the comparative UV dispersion 1 according to Example 1A |
| SPF calc. | | | 15.0 | 15.8 |
| UVA-PF Calc. | | | 5.1 | 13.2 |

### Results - Cell Viability

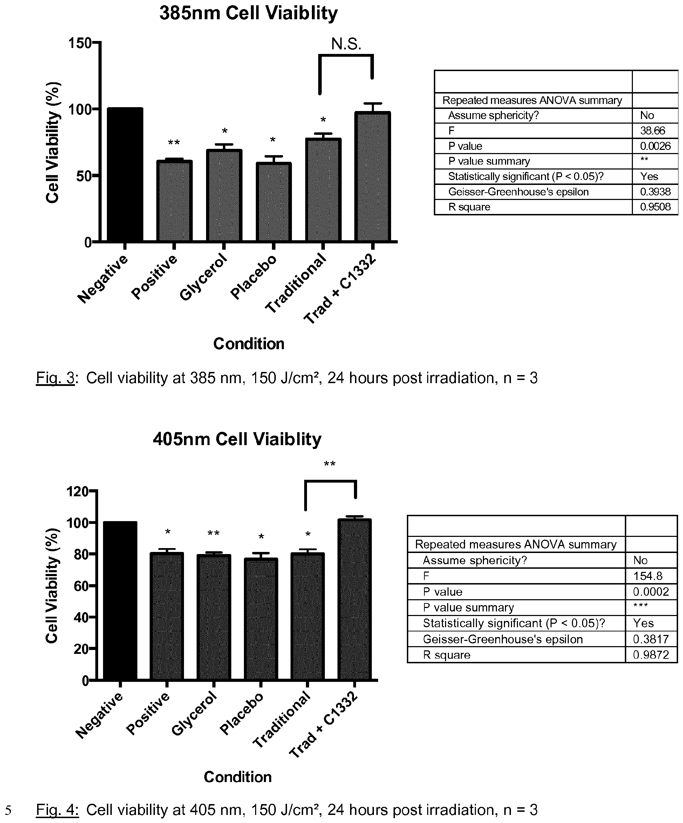

Figures 3 and 4 show the results of cell viability at 385 nm and 405 nm, respectively, both after a dose of 150 J/cm². A linear dose response relationship had been previously shown to exist up to that dose.

The negative control (without irradiation) is set to 100% cell viability. The positive control refers to irradiation without any protection and gives in the case of 385 nm a reading of about 60% cell viability and in the case of 405 nm of 80%. In both cases the cell viability of the negative control can be achieved when using the sunscreen containing compound (1c).

### Results - Reactive Oxygen Species (ROS)

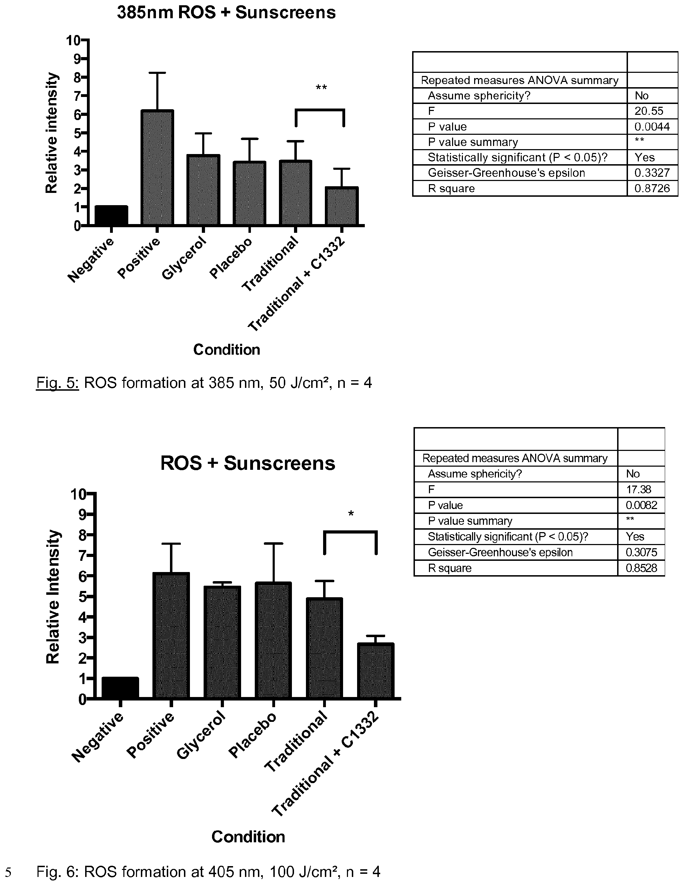

Figures 5 and 6 show the results of ROS formation in the cells at 385 nm after a UV dose of 50 J/cm² and at 405 nm at a light dose of 100 J/cm², respectively. In both cases dose response experiments are carried out previously, showing linear relationships of ROS formation up to the respective dose. The negative control refers to the situation without irradiation, the positive control to that with irradiation without any protection. The best results in terms of suppression of ROS formation are achieved at both wavebands with the sunscreen containing compound (1c).

### Results - Gene Expression Experiments at 385 nm

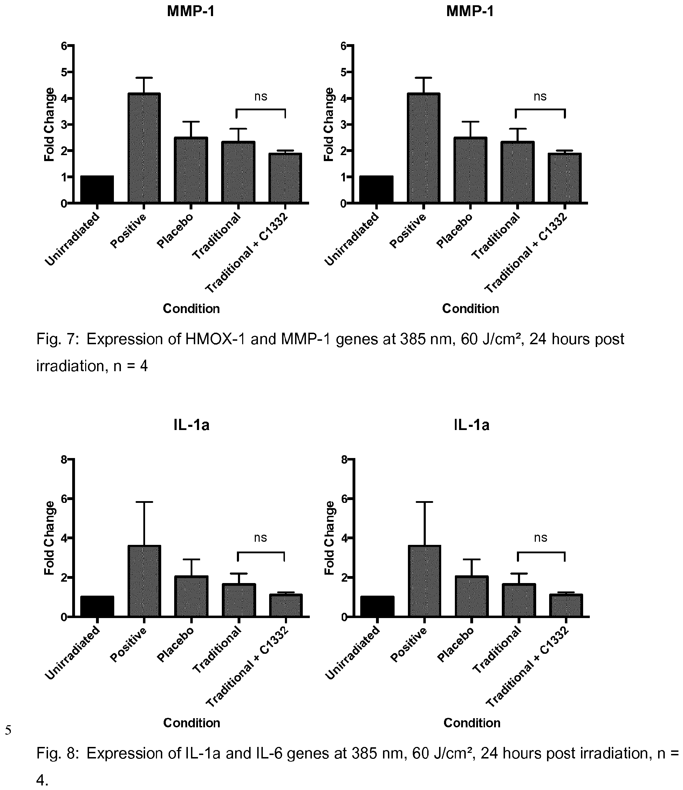

Figures 7 and 8 show gene expression experiments with 385 nm radiation for HMOX-1, MMP-1, IL-1a and IL-6, all involved with skin aging and inflammation processes, all after a dose of 60 J/cm² and 24 hours after termination of irradiation.

The expression of these genes is induced by 385nm radiation and a linear dose-response relationship had been demonstrated previously up to 100 J/cm². In each case the induction is suppressed best by applying the sunscreen containing compound (1c).

### Results - Gene Expression Experiments at 405 nm

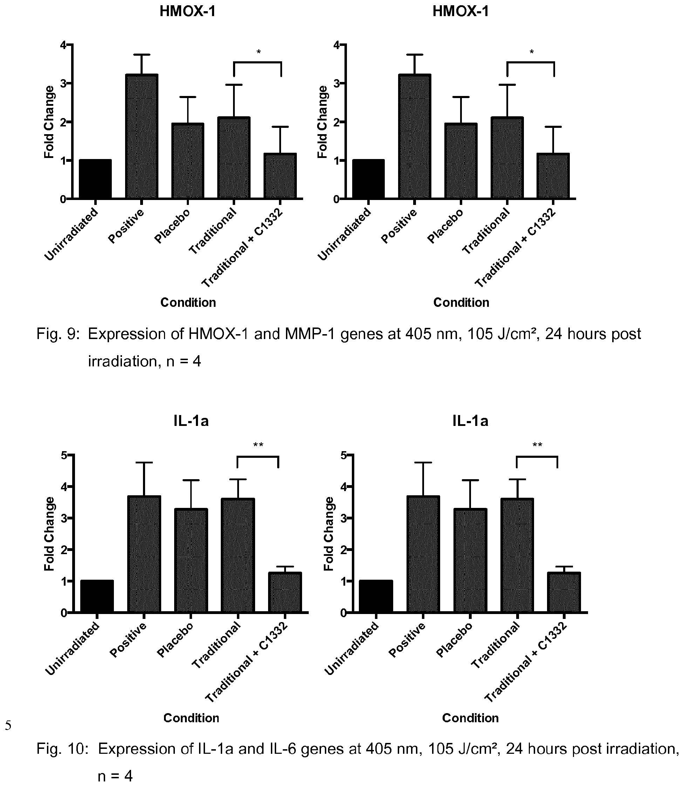

Figures 9 and 10 show gene expression experiments after with 405 nm radiation for HMOX-1, MMP-1, IL-1a and IL-6, all involved with skin aging and inflammation processes, all after a dose of 105 J/cm² and 24 hours after termination of irradiation.

The expression of these genes is induced by 405 nm radiation and a linear dose-response relationship had been demonstrated previously up to 150 J/cm². In each case the induction is suppressed best by applying the sunscreen containing compound (1c).

### Conclusion

The experimental work on keratinocyte cell cultures shows that a sunscreen of SPF 15 containing compound (1c) shows beneficial effects in comparison to a conventional sunscreen of SPF 15. The beneficial effects are related to cell viability, ROS production in the cells and expression of genes related to skin aging and inflammation reactions.

## Claims

1. An aqueous dispersion (B) comprising
20 to 60 % by weight of at least one UV filter selected from
(a₁) the micronized compound of formula 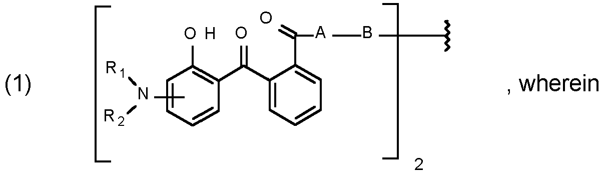
R₁ and R₂ independently from each other are C₁-C₂₀alkyl; C₂-C₂₀alkenyl; C₃-C₁₀cycloalkyl; C₃-C₁₀cycloalkenyl; or R₁ and R₂ together with the linking nitrogen atom form a 5- or 6-membered heterocyclic ring;
A is --N(R₃)-; -O-; or the direct bond;
B is a bivalent radical selected from alkylene, cycloalkylene alkenylene or phenylene radical which is optionally substituted by a carbonyl- or carboxy group; a radical of formula *-CH₂C≡C-CH₂-* ; or the divalent radical *-B-* corresponds to the formula 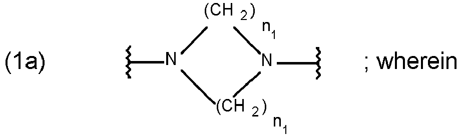
n₁ is a number from 1 to 3;
A is --N(R₃)-; or -O-; and
R₃ is hydrogen; C₁-C₅alkyl; or hydroxy-C₁-C₅alkyl;
(a₂) the micronized compound of formula 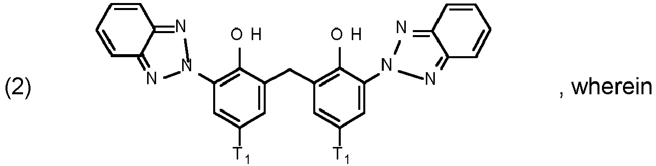 T₁ is hydrogen; C₁-C₁₂alkyl; preferably iso-octyl, or C₁-C₄alkyl substituted by phenyl;
(a₃) the micronized compound of formula 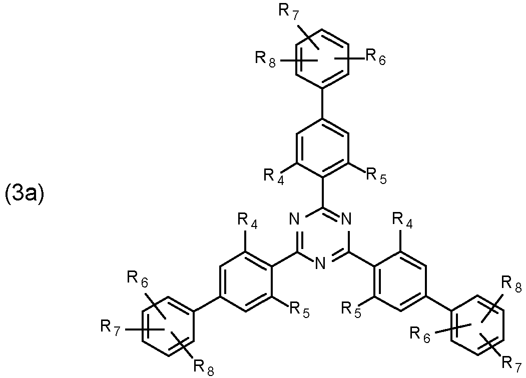 ; wherein
R₄ and R₅ independently from each other are hydrogen; C₁-C₁₈alkyl; or C₆-C₁₂aryl;
R₆, R₇ and R₈ independently from each other, are hydrogen; C₁-C₁₈alkyl; or a radical of formula 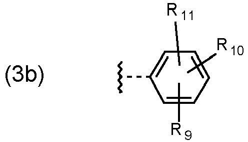 ; wherein
R₉, R₁₀ and R₁₁ independently from each other, are hydrogen; or C₁-C₁₈alkyl; and
(a₄) the micronized compound of formula 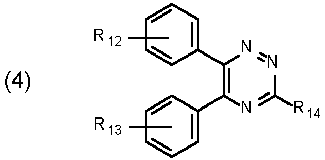 wherein
R₁₂ and R₁₃, independently from each other, represent hydrogen; halogen; C₁-C₁₂alkyl: C₁-C₁₈hydroxy; C₁-C₁₈alkoxy; poly(ethoxy)-alkoxy with a C₁-C₄ alkyl fragment and an ethoxy number ranging from 1 to 4; C₁-C₄mono- or dialkyl amino,
R₁₄ represents halogen; hydroxy; amino; phenyl which is optionally substituted 1 to 3 times by a hydroxy radical situated at least in a para or phenyl position, possibly 1 to 3 times substituted in an ortho, meta or para position by a C₁-C₁₂alkoxy or cyano or C₁-C₇alkylamino group;
0.1 to 20 % by weight of at least one dispersant (c) selected from glutamates;
0 to 1 % by weight of Ingredients based on polydimethylsiloxane/silica; and
ad 100 % water.

2. Aqueous dispersion according to claim 1, characterized that it does not contain any additional thickeners.

3. Aqueous dispersion according to claim 1, characterized that it does not contain thickeners selected from propylene glycol, carbomer and vegetable gums.

4. Aqueous dispersion according to claim 1, characterized that it does not contain Xanthan gum.

5. Sunscreen composition comprising
(i) 0.1 to 50%, preferably 0.5 to 20% by weight, based on the sunscreen composition of the aqueous dispersion (B) as defined in claim 1 and optionally
(ii) a cosmetically acceptable carrier
for the suppression of reactive oxygen species (ROS).

6. Sunscreen composition comprising
(i) 0.1 to 50%, preferably 0.5 to 20% by weight, based on the sunscreen composition of the aqueous dispersion (B) as defined in claim 1 and optionally
(ii) a cosmetically acceptable carrier
for the suppression of induction of gene expression related to skin aging and inflammation reactions.

## Patentansprüche

1. Wässrige Dispersion (B), umfassend
20 bis 60 Gew.-% mindestens eines UV-Filters, ausgewählt aus
(a₁) der mikronisierten Verbindung der Formel 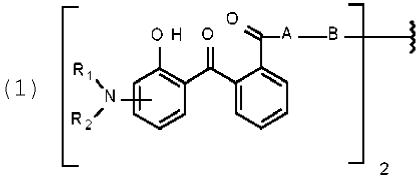 , wobei
R₁ und R₂ unabhängig voneinander für C₁-C₂₀-Alkyl; C₂-C₂₀-Alkenyl; C₃-C₁₀-Cycloalkyl oder C₃-C₁₀-Cycloalkenyl stehen oder R₁ und R₂ zusammen mit dem sie verknüpfenden Stickstoffatom einen 5- oder 6-gliedrigen heterocyclischen Ring bilden;
A für -N(R₃)-; -O- oder die direkte Bindung steht;
B für einen zweiwertigen Rest, der aus einem Alkylen-, Cycloalkylen-, Alkenylen- oder Phenylenrest, der gegebenenfalls durch eine Carbonyl- oder Carboxygruppe substituiert ist, ausgewählt ist; oder einen Rest der Formel 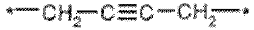 steht oder der zweiwertige Rest *-B-* der folgenden Formel entspricht: 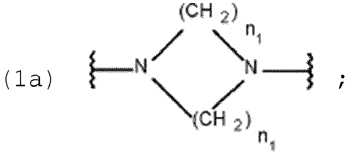 wobei
n₁ für eine Zahl von 1 bis 3 steht;
A für -N(R₃)- oder -O- steht und
R₃ für Wasserstoff; C₁-C₅-Alkyl oder Hydroxy-C₁-C₅-alkyl steht;
(a₂) der mikronisierten Verbindung der Formel 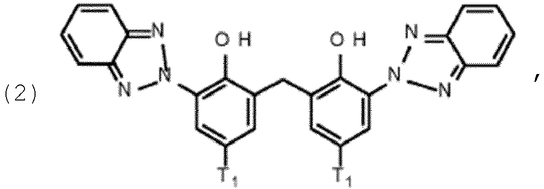 wobei
T₁ für Wasserstoff; C₁-C₁₂-Alkyl; vorzugsweise Isooctyl, oder C₁-C₄-Alkyl, das durch Phenyl substituiert ist, steht;
(a₃) der mikronisierten Verbindung der Formel 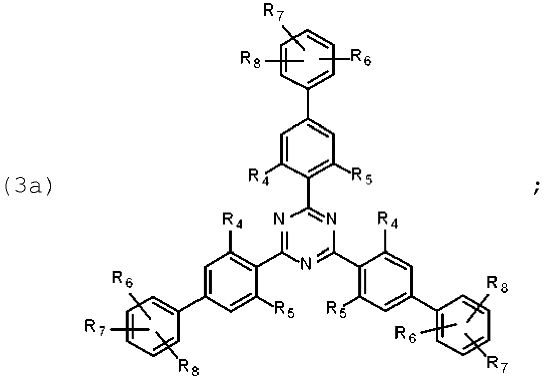 wobei
R₄ und R₅ unabhängig voneinander für Wasserstoff; C₁-C₁₈-Alkyl oder C₆-C₁₂-Aryl stehen;
R₆, R₇ und R₈ unabhängig voneinander für Wasserstoff; C₁-C₁₈-Alkyl oder einen Rest der Formel 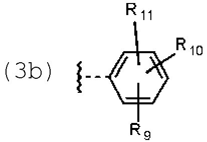 stehen; wobei
R₉, R₁₀ und R₁₁ unabhängig voneinander für Wasserstoff oder C₁-C₁₈-Alkyl stehen; und
(a₄) der mikronisierten Verbindung der Formel 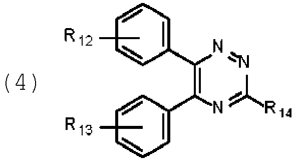 wobei
R₁₂ und R₁₃ unabhängig voneinander für Wasserstoff; Halogen; C₁-C₁₂-Alkyl; C₁-C₁₈-Hydroxy; C₁-C₁₈-Alkoxy; Poly(ethoxy)alkoxy mit einem C₁-C₄-Alkylfragment und einer Ethoxyzahl im Bereich von 1 bis 4 oder C₁-C₄-Mono- oder -Dialkylamino stehen,
R₁₄ für Halogen; Hydroxy; Amino; Phenyl, das gegebenenfalls 1- bis 3-fach durch einen Hydroxyrest substituiert ist, der zumindest in einer para- oder Phenylposition steht, gegebenenfalls 1- bis 3-fach in einer ortho-, meta- oder para-Position durch eine C₁-C₁₂-Alkoxy- oder Cyano- oder C₁-C₇-Alkylaminogruppe substituiert ist, steht;
0,1 bis 20 Gew.-% mindestens eines Dispergiermittels (c), das aus Glutamaten ausgewählt ist;
0 bis 1 Gew.-% Inhaltsstoffe auf Basis von Polydimethylsiloxan/Siliciumdioxid und
ad 100 % Wasser.

2. Wässrige Dispersion nach Anspruch 1, **dadurch gekennzeichnet, dass** sie keine zusätzlichen Verdicker enthält.

3. Wässrige Dispersion nach Anspruch 1, **dadurch gekennzeichnet, dass** sie keine Verdicker enthält, die aus Propylenglykol, Carbomer und pflanzlichen Gummen ausgewählt sind.

4. Wässrige Dispersion nach Anspruch 1, **dadurch gekennzeichnet, dass** sie kein Xanthangummi enthält.

5. Sonnenschutzzusammensetzung, umfassend
(i) 0,1 bis 50 Gew.-%, vorzugsweise 0,5 bis 20 Gew.-%, bezogen auf die Sonnenschutzzusammensetzung, der wässrigen Dispersion (B) gemäß Anspruch 1 und gegebenenfalls
(ii) einen kosmetisch unbedenklichen Träger,
zur Unterdrückung reaktiver Sauerstoffspezies.

6. Sonnenschutzzusammensetzung, umfassend
(i) 0,1 bis 50 Gew.-%, vorzugsweise 0,5 bis 20 Gew.-%, bezogen auf die Sonnenschutzzusammensetzung, der wässrigen Dispersion (B) gemäß Anspruch 1 und gegebenenfalls
(ii) einen kosmetisch unbedenklichen Träger,
zur Unterdrückung der Induktion von Genexpression im Zusammenhang mit Hautalterung und Entzündungsreaktionen.

## Revendications

1. Dispersion aqueuse (B) comprenant
20 à 60 % en poids d'au moins un filtre UV choisi parmi
(a₁) le composé micronisé de formule 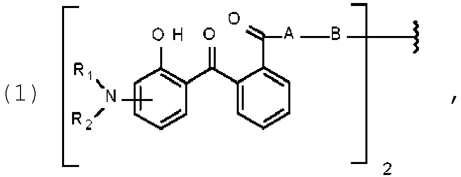
R₁ et R₂ indépendamment l'un de l'autre étant C₁-C₂₀alkyle ; C₂-C₂₀alcényle ; C₃-C₁₀cyclo-alkyle ; C₃-C₁₀cycloalcényle ; ou R₁ et R₂ conjointement avec l'atome d'azote de liaison formant un cycle hétérocyclique à 5 ou 6 chaînons ;
A étant -N(R₃)- ; -O- ; ou la liaison directe ;
B étant un radical bivalent choisi parmi un radical alkylène, cycloalkylène alcénylène ou phénylène qui est éventuellement substitué par un groupe carbonyle ou carboxy ; un radical de formule *-CH₂-C≡C-CH₂-* ; ou le radical divalent *-B-* correspondant à la formule 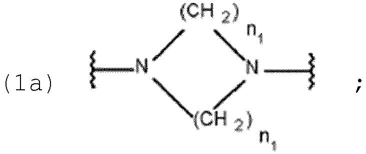
n₁ étant un nombre de 1 à 3 ;
A étant -N(R₃)- ; ou -O- ; et
R₃ étant hydrogène ; C₁-C₅alkyle ; ou hydroxy-C₁-C₅alkyle ;
(a₂) le composé micronisé de formule 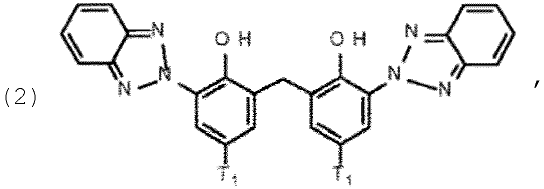 T₁ étant hydrogène ; C₁-C₁₂alkyle ; préférablement iso-octyle, ou C₁-C₄alkyle substitué par phényle ;
(a₃) le composé micronisé de formule 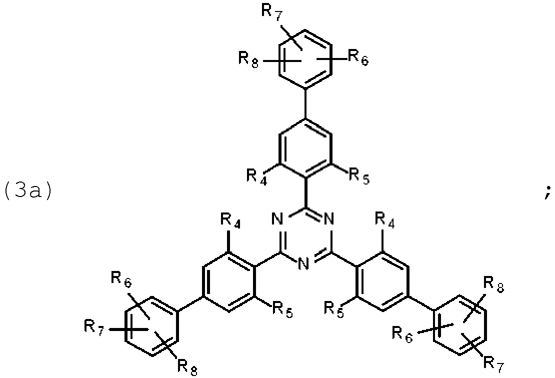
R₄ et R₅ indépendamment l'un de l'autre étant hydrogène ; C₁-C₁₈alkyle ; ou C₆-C₁₂aryle ;
R₆, R₇ et R₈ indépendamment les uns des autres, étant hydrogène ; C₁-C₁₈alkyle ; ou un radical de formule 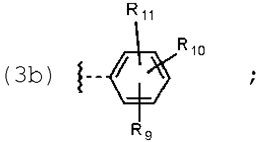
R₉, R₁₀ et R₁₁ indépendamment les uns des autres, étant hydrogène ; ou C₁-C₁₈alkyle ; et
(a₄) le composé micronisé de formule 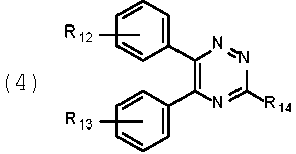
R₁₂ et R₁₃, indépendamment l'un de l'autre, représentant hydrogène ; halogène ; C₁-C₁₂alkyle ; C₁-C₁₈hydroxy ; C₁-C₁₈alcoxy ; poly(éthoxy)-alcoxy comportant un fragment C₁-C₄alkyle et un nombre d'éthoxy dans la plage de 1 à 4 ; C₁-C₄mono- ou dialkyle amino,
R₁₄ représentant halogène ; hydroxy ; amino ; phényle qui est éventuellement substitué 1 à 3 fois par un radical hydroxy situé au moins dans une position para ou phényle, possiblement 1 à 3 fois substitué dans une position ortho, méta ou para par un groupe C₁-C₁₂alcoxy ou cyano ou C₁-C₇alkyl-amino ;
0,1 à 20 % en poids d'au moins un dispersant (c) choisi parmi des glutamates ;
0 à 1 % en poids d'ingrédients à base de polydiméthylsiloxane/silice ; et
ad 100 % d'eau.

2. Dispersion aqueuse selon la revendication 1, **caractérisée en ce qu'**elle ne contient aucun épaississant supplémentaire.

3. Dispersion aqueuse selon la revendication 1, **caractérisée en ce qu'**elle ne contient pas d'épaississants choisis parmi le propylène glycol, un carbomère et des gommes végétales.

4. Dispersion aqueuse selon la revendication 1, **caractérisée en ce qu'**elle ne contient pas de gomme de xanthane.

5. Composition d'écran solaire comprenant
(i) 0,1 à 50 %, de préférence 0,5 à 20 % en poids, sur la base de la composition d'écran solaire de la dispersion aqueuse (B) telle que définie dans la revendication 1 et éventuellement
(ii) un support acceptable sur le plan cosmétique pour la suppression d'espèces d'oxygène réactif (ROS).

6. Composition d'écran solaire comprenant
(i) 0,1 à 50 %, de préférence 0,5 à 20 % en poids, sur la base de la composition d'écran solaire de la dispersion aqueuse (B) telle que définie dans la revendication 1 et éventuellement
(ii) un support acceptable sur le plan cosmétique pour la suppression de l'induction de l'expression génique liée au vieillissement cutané et aux réactions d'inflammation.
